(19) Europäisches Patentamt
European Patent Office
Office européen des brevets

(11) EP 4 696 224 A1

(12) EUROPEAN PATENT APPLICATION

(43) Date of publication:
18.02.2026 Bulletin 2026/08

(21) Application number: 24194607.8

(22) Date of filing: 14.08.2024

(51) International Patent Classification (IPC):
A61B 5/00 (2006.01)   A61N 2/00 (2006.01)
A61B 5/02 (2006.01)   A61N 5/10 (2006.01)
G16H 30/40 (2018.01)   G16H 40/63 (2018.01)
G16H 50/20 (2018.01)

(52) Cooperative Patent Classification (CPC):
A61B 5/0037; A61B 5/004; A61B 5/6892;
A61B 5/70; A61N 5/1049; G16H 30/40;
G16H 40/63; G16H 50/20; A61B 5/02;
A61B 2562/0247

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Koninklijke Philips N.V.
5656 AG Eindhoven (NL)

(72) Inventors:
• SENEGAS, Julien Thomas
Eindhoven (NL)

• DENISSEN, Adrianus Johannes Maria
Eindhoven (NL)
• VAN EE, Raymond
5656AG Eindhoven (NL)
• WIEMKER, Rafael
Eindhoven (NL)
• KRUEGER, Sascha
Eindhoven (NL)

(74) Representative: Philips Intellectual Property &
Standards
High Tech Campus 52
5656 AG Eindhoven (NL)

(54) **IMPROVED PREDICTION OF SCAN POSITION OF INTERNAL ORGANS**

(57) System (FS) and related method for localizing an object of interest (OI) for medical imaging. The system comprises an input (IN) interface through which is receivable input data comprising a spatial map of non-line-of-sight sensor readings (s) from plural sensors (NLS, Nij)) collectable in respect of a patient (PAT). A spatial characterizer (SC, LC) of system determines, based on said input data, a position ($p$) of said object of interest (OI). The input data includes further data ($m$) providable by a line-of-sight based sensor (LS) in relation to the patient (PAT).

Fig. 3

EP 4 696 224 A1

**Description**

FIELD OF THE INVENTION

The invention relates to system for localizing object of interest for medical imaging, to an imaging arrangement including such as system, to a related method, to a machine learning training system for training a machine learning model for use in such a system, to a training data provider system for providing training data for training the model, to related methods of training such machine learning model and for providing training data for such training, to a computer program element, and to a computer readable medium.

BACKGROUND OF THE INVENTION

Proper positioning of a target anatomy of a patient with respect to an imaging system (eg, CT, MR) is a consideration to ensure "first-time right" imaging, to optimize image quality for diagnostic support, to optimize signal to noise ratio ("SNR") and, in X-ray exams, dose distribution.

Positioning may include horizontal positioning, which typically defines the field-of-view for a localizer or diagnostic scan, and vertical positioning, which ensures proper centering of the target anatomy.

Nowadays, the selection of horizontal and vertical positions can be automated by means of camera-based approaches, where the position of the target anatomy (object of interest) is computed by processing camera images. However, accurate models are needed to predict the position of the target anatomies based on such camera imagery. What is more, current purely camera-imaging based approaches for automated or computer-assisted positioning rely on the definition of outside anatomical features in the imagery. Such anatomical features, typically based on clinical guidelines, need to be within the line-of-sight of the camera system. This typically allows selecting the isocenter of the localizer or diagnostic scan (for MRI), or of start and end points of the localizer or diagnostic scan (for CT), based on external landmarks, such as chin, shoulders, sternum, elbows, hips, etc. Computer vision processing may be used to identify such landmarks.

However, the accurate prediction from such external imagery of the position of an internal object of interest, such as of the liver, the lung diaphragm, or the heart, may be difficult due to high variability amongst subjects. This in in particular because the position of some such internal objects of interest (organs, etc) can differ significantly within subjects with similar outside morphology.

Another limitation of purely camera-based positioning is posed by occlusions. The purely camera-based systems may experience difficulties in estimating anatomical landmarks when there is line-of -sight due to occlusion, e.g. when medical personnel steps in between the camera and patient, when there are blankets, etc, or other occluding objects.

WO 2021/256921A1 describes a two-dimensional pressure sensing system.

SUMMARY OF THE INVENTION

There may therefore be a need for improved approaches for patient positioning in imaging, in particular in medical imaging.

An object of the present invention is achieved by the subject matter of the independent claims, wherein further embodiments are incorporated in the dependent claims. It should be noted that the following described aspect of the invention equally applies to the related method, to the machine learning training system for training the machine learning model for use in such as system, to the training data provider system for providing training data for training the model, to the related methods of training such machine learning model and for providing the training data for such training, to the computer program element, and to the computer readable medium.

According to a first aspect of the invention there is provided a system for localizing an object of interest for medical imaging, comprising:

> an input interface configured for receiving input data comprising i) a spatial map of non-line-of-sight sensor readings from plural sensors
> collectable in respect of a patient, and ii) further data providable by a line-of-sight based sensor in relation to the patient; and
> a spatial characterizer configured for determining, based on said spatial map of non-line-of-sight sensor readings data and on the said further data, a current position of said object of interest.

The determined position may be relative to a reference coordinate system.

In embodiments, the said sensors are arrangeable in a 2D (two-dimensional) pattern.

The spatial map is formed from non-line-of sight senor readings. Preferably, and in some embodiments, the readings form spatio-temporal data. Thus, some or each entry in the map corresponds to a respective position of a respective one of the plural sensors, and each such entry is such a senor reading at that position, subject to changes over time, thus defining the spatio-temporal map. Thus, the map may be considered a collection of spatially resolved time series of sensor readings. The map may be stored as an entity (such as in a matrix). However, in some embodiments, the non-line of sight senor readings are static, akin to a snapshot.

In embodiments, the said sensors are at least partly integrated in a patient support of a medical imaging apparatus.

In embodiments, the said sensors are capable for collecting such readings across the patient in at least two spatial dimensions.

In embodiments, the said sensors are capable of collecting such readings for the patient as a whole.

The spatial characterizer may operate on "dual data" input including the spatial map of non-line-of-sight sensor readings from plural sensors and the further data providable by the (at least one) line-of-sight based sensor.

Specifically, the spatial characterizer may be operable to combine information from this dual data. Thus, the spatial characterizer may be operable to combine data from the spatial map of non-line-of-sight sensor readings and from the said further data providable by the line-of-sight based sensor.

Operating on such dual data has been found to improve reliability and/or accuracy of the determined position. One data type in the dual data may be used to regularize the other.

Various manners of combing such dual data is envisaged herein.

In embodiments, the spatial characterizer is based on a trained machine learning model. Thus, in such embodiments, such dual data is fed into the machine learning model and is co-processed there.

In some embodiments, the combining may include forming and analyzing correlation between the line of sight data and the non-line of sight data.

In embodiments, the spatial characterizer capable of time-series waveform analysis or of spectral analysis of the said readings. Specifically, in embodiments, the spatial characterizer is capable of time-series waveform analysis or of spectral analysis of the said readings, based on the line of sight data. Yet more specifically, and in embodiments, the processing of the non-line of sight readings may be constrained by the line of sight data, or the other way around. For example, based on the line of sight data, a (spatial subset) of such readings are identified, and the spatial characterizer then analysis only readings from the said subset to determine the position. This allows reducing computational burden as fewer data points may be considered.

In embodiments, the spatial characterizer is capable of isolating in the map sensor readings a signal representative of an activity attributable to the object of interest, or to a surrogate object spatially related to the object of interest.

In embodiments, the signal incudes a physiological signal.

For example, the physiological signal may be representative of a cardiac or breathing motion, and based on this, the source of the cardiac or breathing activity (eg, motion) is determined to improve the computation of object of interest (in this case, heart or lung(s)).

Thus, in some embodiments, the spatial characterizer is capable of spatio-temporal analysis of the map. In some embodiments, for a given spatio-temporal reading as per the map, it is determined whether this reading is related to the object of interest. This is done for some or each readings from different respective sensors of the plural sensors. The positions of all sensors whose readings are so found to be related to the object of interest then together define any one or more of position, spatial extent/size, shape of object of interest, or distance from the said object to other object(s)/landmarks. The spatio-temporal analysis is either explicit (such as in some time series analysis embodiments), or is implicit such as in some end-to-end machine learning embodiments, or other.

In embodiments, the spatial characterizer is capable of determining a size (spatial extent) of the object of interest and/or a distance between said object of interest and another object of interest or landmark.

The input data includes the said further data providable by the line-of-sight based sensor in relation to the patient. Thus, in preferred embodiments, the input data has dual character as it includes the said non-line-of-sight sensor readings, in addition to line-of-sight data as provided by the line-of-sight sensor. The combination of both, non-line-of-sight data and line-of-sight data, has been found to be of benefit herein, as one such data set may complement a possible deficiency of the other data set. For example, a visual occlusion, which may affect the line-of-sight data, may be at least partially compensated for by the non-line of sight data.

In embodiments, the said line-of-sight based sensor includes a camera. This may be a camera for still or video imagery. The camera, video or still, may be an optical camera, color camera, infra-red camera, depth camera, thermal camera.

In embodiments, the system further comprises a control interface capable to effect any one of more of: adjusting an imaging geometry of an imaging apparatus, and triggering an imaging operation. In general, imaging geometry relates to the spatial configuration (position/and or orientation) between object of interest, source and/or detector of imager. Thus, such adjustment may include moving the patient support (with object of interest on it) to new position, based on determined position.

In another aspect there is provided an arrangement comprising a system of any one of preceding claims, and further comprising any one of more of: i) a display device for displaying any one of: the determined position, size or distance ii) any one or more of the said sensors, iii) the, or a, patient support, iv) the imaging apparatus, v) an imaging geometry adjuster capable of adjusting an imaging geometry of the, or a, imagining apparatus, based on the said current position ($p$) of said object of interest.

In another aspect there is provided the training system for training, based on training data, the ML model.

In another aspect there is provided a training data provider capable of providing the training data for training of model by the training system.

In another aspect there is provided a method for localizing an object of interest for medical imaging, comprising:

receiving input data comprising i) a spatio-temporal map of non-line-of-sight sensor readings from plural sensors collectable in respect of a patient and ii) further data providable by a line-of-sight based sensor in relation to the patient; and

determining, based on said spatio-temporal map of non-line-of-sight sensor readings (s) and on the said further data, a current position of said object of interest.

In another aspect there is provided a method of training, based on training data, the machine learning model.

In another aspect there is provided a method of providing training data for training the model.

In another aspect there is provided a computer program element, which, when being executed by at least one processing unit, is adapted to cause the processing unit to perform the method for facilitating object of interest positioning for medical imaging, or the method of training, or the method of providing training data.

In another aspect there is provided at least one computer readable medium having stored thereon the program element or having stored thereon the trained machine learning model.

It has been found herein that certain (internal) object of interest (eg organs, such as the heart, lungs, etc) exhibit activity (such as motion of any kind) and that this activity may be reflected, eg by modulation, in certain patterns of the non-line-of-sight spatio-temporal measurement readings. In some embodiments, the said activity is physiological, but may also include other activity, such as a motion of a limb, whether done voluntarily or involuntarily, such as a tremor of arm leg, or head. Also, the said, activity, whether or not physiological in nature, may relate to a surrogate object, different from the object of interest, but may be in a spatial relation to the object of interest. In the following however, main reference will be made to the physiological nature of the data, and such data will be referred to as "physiological data", with the understanding the below described is of equal application to the broader concept of spatio-temporal non-line-of-sight data that is capable of being modulated by activity, whether or not such activity is physiological or not.

The non-line-of-sight measurement readings may thus be understood to supply in some embodiments spatio-temporal physiological data which can be used by the proposed system to spatially characterize object of interest, such as its position in the examination region/image domain of the imagery, and optionally any one or more of: its shape, size and distance to one or more externally visible landmarks. In case of the surrogate object, its activity may be measured instead by the non-line-of-sight spatio-temporal readings but can still be used to determine the spatial characteristics (eg, position) of the object of interest, due to a known spatial relationship, or by machine learning.

Whilst using such physiological data on its own can be used for such spatial characterization, in some embodiments it has been found to be useful to combine spatio-temporally measurements of physiology data provided by the one or more non-line-of-sight sensor, with morphology information provided by another sensor, such as a line-of-sight sensor, in order to improve the accuracy of the prediction of the spatial characterization of the internal object of interest. Especially, what is proposed herein is to use spatio-temporal signals from a pressure sensing mattress positioned between the subject and the patient support in combination with an overhead camera system.

Such pressure sensing mattress or any other pressure sensing system capable of spatio(distributed)-temporal sensing is placed below the subject's body. Such pressure sensor system may for example be integrated within a mattress positioned on top of the patient support. Each node of the pressure sensing mattress matrix delivers a temporal signal representative of motion and physiology, such as respiration and cardiac beating. The temporal signal of each node is processed to identify whether this signal corresponds to a respiratory signal, a cardiac signal or one of the static or aperiodic, main body pressure point. For example, pressure signal may peak statically or un-periodically for individual nodes and may thus be taken as indicative of a backside position of the anatomy of interest. Typical such examples include the main body pressure points such as head, feet, elbow, hip and shoulders. For example, this can be achieved by frequency analysis of the temporal signals or machine learning. As a result, the location of the nodes bearing e.g. the strongest respiration or cardiac signals can be extracted and used to spatially characterize the object of interest (eg, heart, lung, diaphragm, etc). What is proposed herein is that such physiological information is combined with morphological information, such as the location of anatomical landmarks/anatomical key points, derived from another sensor, such as an overhead camera. In this dual-stream (physiological + morphological data)-approach, the position of object(s) of interest, such as lung diaphragm, heart and liver can be obtained with improved accuracy. For example, a regression model taking as input the locations of the pressure sensing nodes and of the anatomical landmarks can be applied. More generally, the correspondence between position of internal organs and signal response of the pressure sensing mattress nodes, also in combination with anatomical data extracted from an overhead camera, can be learned via a machine learning, such as deep learning methods or other. The proposed system delivers meaningful data even in cases where the view on patient is at least partially occluded, and, equally, can improve systems, such as described in WO 2021/256921A1, that solely rely on non-line of sight data, thanks to the proposed dual data setup.

The proposed setup can be used for MR, CT / nuclear imaging such as PET/SPECT, and Digital X-ray radiogrammetry ("DXR") applications. It especially applies to systems equipped with an automated set-up system such as an overhead camera and a patient support.

"*user*" relates to a person, such as medical personnel or other, operating the imaging apparatus or overseeing the imaging procedure. In other words, the user is in general not the patient.

The terms *"object of interest", "region of interest"* of *"(target) anatomical feature"* will be used herein interchangeably. This relates to the actual part of the human body that is the subject or purpose of the imaging, in particular of the diagnostic scan/high dose projection data acquisition. Such object of interest may be located within the patient's body. The term *"object of interest"* as used herein may refer to any at least partly internal organ, group of organs, part of a organ(s), tissue type(s), etc. The term *"object of interest"* as used herein may also be understood to refer to foreign objects in the body (if any), such as implants, ingested material, etc. The term may also be applicable in non-medical applications, such a baggage scanning, non-destructive material testing, etc. Object of interest may be mostly within human body, but may be located outside. However, in the latter case it may be adherently or inadvertently covered/occluded by objects such as blankets, cloths, hospital gowns, etc.

*"surrogate object"* may be generator of activity, instead of the object of interest. Like the object of interest, surrogate object may be organ, part thereof, group of organs, etc. Thus, surrogate object may not be the (actual) object of interest and the purpose of imaging, so long as there is a known or learnable spatial relationship between object of interest OI and surrogate object. Surrogate object may within human body, or outside. Surrogate object may cause activity of the object of interest, such as the heart activity causing slight motion of the liver, such as in liver imaging, or similar configurations. In such cases when the surrogate object causes, that is, induces, the activity of the object of interest, the non-line-of-sight sensors may then pick up the induced activity in the measured non-line-of-sight readings. Thus, the activity of the object of interest as measured may be an induced one.

*"activity"* as used herein may pertain to object of interest or to surrogate object. The activity may or may not be physiological. If the activity is one of the object, this may induced by the surrogate object, and thus may not be physiological. The non-line of sight sensor readings as per the map may pertain to the activity of the object of interest, whether or not the activity is induced.

*"physiological "* relates to activity/phenomenon, state, etc, as may be caused during normal or pathological operation of human body.

*"positioning"* of object of interest refers to localizing the object in image domain/examining region/bore of imager, and, once localized, adjusting, if needed, an imaging geometry of the imager for improved imaging results, such as according to imaging protocol.

In general, the term *"machine learning"* includes a computerized arrangement (or module) that implements a machine learning ("ML") algorithm, but may more broadly relate to any setup for learning a task from data. Some such ML algorithms operate to adjust a machine learning model that is configured to perform (thus, "learn") the task. Other ML operate direct on training data, in which case the training data may form (at least part of) the model. This adjusting or updating of the model is called "training". In general task performance by the ML module may improve measurably, with training experience. Training experience may include suitable training data and exposure of the model to such data. Task performance may improve the better the data represents the task to be learned. Training experience helps improve performance if the training data well represents a distribution of examples over which the final system performance is measured. See for example, T. M. Mitchell, "Machine Learning", page 2, section 1.1, pages 5-6, section 1.2.1, McGraw-Hill, 1997. The performance may be measured by objective tests based on output produced by the model in response to feeding the model with test data. The performance may be defined in terms of a certain error rate to be achieved for the given test data.

BRIEF DESCRIPTION OF THE DRAWINGS

Exemplary embodiments of the invention will now be described with reference to the following drawings, which, unless stated otherwise, are not to scale, wherein:

Fig. 1 shows a schematic diagram of a medical imaging arrangement;
Figs 2 show, in a schematic manner, positioning considerations in relation to an object to be imaged by an imaging apparatus;

Fig. 3 shows a block diagram of a facilitator system capable of facilitating an imaging operation;

Figs 4 illustrate a layout of non-line-of-sight sensors;

Fig. 5 shows control circuitry as may be used in the sensor layout of Fig. 4;

Fig. 6 shows examples of time domain curves of non-line-of-sight sensor readings in comparison with a time domain physiological signal;

Fig. 7 shows further examples of time domain curves of non-line-of-sight sensor readings in comparison with a time domain physiological signal;

Figs 8 illustrate of a line-of-sight sensor arrangement;

Fig. 9 shows a flow chart of a computer-implemented method of facilitating imaging, in particular facilitating localization in relation of an object of interest to be imaged;

Fig. 10 shows a block diagram of a machine learning model as may be used herein in embodiments;

Fig. 11 shows a block diagram of a training system as may be used herein to train a machine learning model on training data as envisaged herein in embodiments;

Fig. 12 shows a flow chart of a computer-implemented method of obtaining training data for training a machine learning model; and

Fig. 13 shows a flow chart of a computer-implemented method of training a machine learning model.

DETAILED DESCRIPTION OF EMBODIMENTS

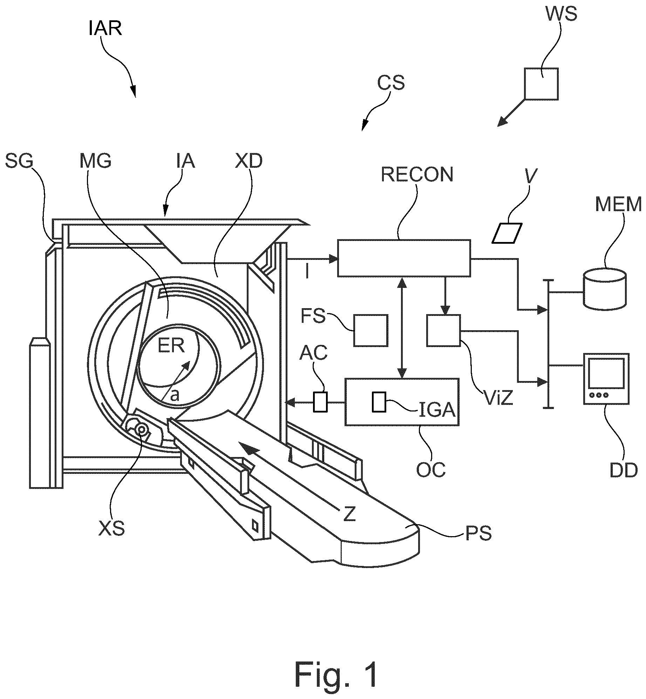

Reference is first made to Fig. 1 which shows a schematic block diagram of a medical imaging arrangement IAR as envisaged herein in embodiments. The arrangement IAR may include a medical imaging apparatus IA ("imager" for short), preferably of the tomographic X-ray based type. Thus, the imager IA may be a computed tomography (CT) scanner, but interventional systems such as those of the C-arm/U-arm-type are not excluded herein. Other tomographic modalities such as MRI, PET and others still are also not excluded herein in some other embodiments. Also, whilst tomography is mainly envisaged herein, radiography (projection only imaging) is not excluded herein.

The arrangement IAR further includes a computing system CS which is broadly operable to process data, including image data, as provided by the imager IA. Generally, the computing system CS supports an imaging (operation) carried out by imager IA. The computing system may further allow controlling operation of the imager. The computing system may be remotely located from the imaging apparatus IA, or may located close thereto, such as integrated into an operator console CS. The computing system allows a user to operate the imaging apparatus on-site or remotely. In particular, user can control an imaging operation to obtain medical imagery for diagnosis, treatment, planning (such as in radiation therapy, or other), etc. Autonomous imaging is also envisaged herein.

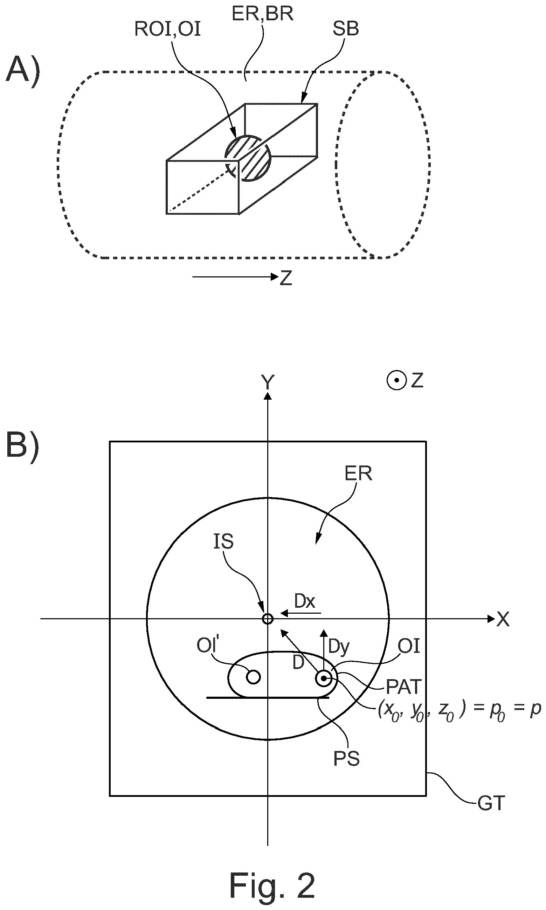

Broadly, and as will be explained more fully below, the computing system CS may include a facilitator system FS that may be used to facilitate obtaining spatial planning data, in particular including positioning data for an object of interest OI to be imaged. The planning data may be used in a diagnostic scan or preliminary scan (locator scan) to acquire sectional or volume data $V$ of the object of interest OI, such as a target anatomical feature, or any other. The planning data may include definition of a scan box SB, a concept described later below at Figs 2. Broadly, the scan box SB is defined by imaging geometry of the imager IA. The scan box SB is data that allows controlling imager IA to acquire, at the right imaging geometry, the right diagnostic tomographic raw data, such as projection data, at a correct spatial position where the object of interest OI is located.

The computing system CS, in particular facilitator system FS, may be implemented as a Cloud solution, that is run on one or more servers or other one or more computational entities. The imaging apparatus IA may be installed in a clinical facility, such as in a hospital for example. The computing system may be installed or used in a control room next to the imaging room where the imager IA is located. In some embodiments the computing system may be integrated into the imager IA. The imager IA may be communicatively coupled via a wired or wireless (or partly both) communication channel CC to computing system CS. The computing system CS may be arranged as a stationary computing system, such as a desktop computer, or as said server, or may be arranged as a mobile device, such as a laptop, smartphone, tablet, etc. For example, the computing system CS may be arranged on a work station WS associable with the imager IA.

Before explain operation of the facilitator system FS in more detail, reference is first mode to components of the imager IA, to so aid the later explanations in relation to the facilitator system FS.

The imaging apparatus IA is operable to generate, in particular acquire, projection data $\lambda$. Once acquired, the projection data $\lambda$ may be forwarded through one or more data communication channels to the computing system CS for reconstruction into tomographic (sectional) image(s) V. The computing system CS may run a reconstructor unit RECON, which implements a tomographic reconstruction algorithm. In general, reconstruction algorithm implement a mapping that maps projection data $\lambda$ located in projection domain into image domain. Image domain is a portion of 3D space and is located in the examination region ER of the imaging apparatus, whilst the projection domain is in general 2D, and is located at an (X-ray) detector XD of the imaging apparatus IA.

The image domain is conceptually made up of a 3D grid of 3D points (voxels), each with its own 3D coordinate v(x,y,z) relative to a reference 3D imaging coordinate system $(X,Y,Z)$ with an origin, which is assumed herein to be defined and given. Such reference coordinate-system with such origin is usually set up when the imager IA is installed in a calibration procedure. Some of the voxel positions v are populated by the reconstruction algorithm with values to so build up the volume V.

As said, the imaging apparatus IA is preferably of the tomographic type, and is preferably configured for multi-directional raw data acquisition , such as projection image acquisition. The imaging apparatus IA is thus capable of acquiring projection images λ along different projection directions α relative to the examination region ER, and thus to the target anatomical feature TAF of /in patient PAT. Acquisition may be done in embodiments by a rotational system where at least the X-ray source XS is arranged in a moveable gantry MG.

The movable gantry MG (and with it, in embodiments, the X-ray source XS) is rotatable in a stationary gantry SG about the examination region ER, in which the patient/ROI resides during imaging. Opposite the X-ray source in the movable gantry there is the X-ray detector XD which may rotate with the gantry and X-ray source around the examination region ER to realize the different projection directions α. The source XS and the detector XD may be collectively referred to the imager IA's acquisition equipment.

As schematically indicated in Fig. 1, patient's longitudinal axis Z (also called imaging axis) may extend into the examination region ER during imaging. Patient PAT may lie on a patient support PS, such as a bed, which is positioned at least partly in the examination region ER during imaging. In some, but not all embodiments, helical imaging protocols are envisaged herein where there is a relative lateral motion along the longitudinal axis Z between X-ray source XS and the patient PAT. For example, the patient support PS may be advanced in a lineal motion through the examination region ER during the multi-directional projection image acquisition, for example during rotation of the X-ray source XS around the patient.

The CT scanner setup as illustrated in the Fig. 1 is merely according to one embodiment, and other tomographic imaging equipment, such as C-arm or U-arm scanners, Cone beam CT arrangements, mammographic imagers, etc, are not excluded herein. C-arm cone beam imagers may be used in some embodiments. In addition, the multi-directional acquisition capability may not necessarily result from a rotational system such as shown in Fig. 1. Non-rotational imaging systems are also envisaged, such as in CT scanners of $4^{th}$ or $5^{th}$ generation, where multiple X-ray sources are arranged around the examination region in a source annulus for example. In addition or instead, the detector XD may be arranged as a detector annulus around the examination region. Thus, in such systems there is no mechanical rotation of the X-ray source XS or detector XD, or of both.

As briefly mentioned above, operator console OC allows user, such as medical personnel, to control the imaging operation. For example, user may request initiating the image acquisition or may request reconstruction or other operations, or may initiate transmission of data to the computing system CS, or may halt such transmission as required. The operator console OC may have integrated therein a display device for viewing control parameters, etc.

During imaging, a respective X-ray beam XB issues forth, along the different projection directions α, from a focal spot of the X-ray source(s) XS. The respective beam XB passes through the examination region with the patient in it. The x-radiation interacts with patient tissue. The X-ray beam XB is modified as a result of this interaction. In general, such X-ray beam XB modification includes attenuation and scattering of the original incoming X-ray beam. The modified X-radiation is then detected as a spatial distribution of various intensities at X-ray sensitive pixels of the detector XD.

It is not necessary herein to acquire projection images λ over a full 360° angular range around the examination region ER. Acquisition over a partial angular range such as a range of 270°, 180°, or even less may be sufficient. The X-ray detector is preferably configured for acquisition of 2D projection images with rows and columns of intensity values as registered by the detector pixels. That is, the detector pixels themselves may be arranged in a matrix layout. Such a 2D layout may be used in divergent imaging geometries, such as cone or fan beam or others. However, one dimensional detector pixel layouts (such as along a single line) are not excluded herein, and neither are parallel beam geometries.

The reconstructor RECON implements one or more reconstruction algorithms to process the diagnostic projection imagery λ. Specifically, the reconstructor RECON may compute tomographic target image V of the examination region (with the patient in it) for diagnostic, therapeutic or other purposes. The reconstructor RECON may be able to produce sectional volumetric image data ("image volume(s)") V. However, this does not exclude producing a single image slice in the examination region as required. Thus, the reconstructed imagery may be denoted herein as V which may include the whole volume, a partial volume or a specific section therethrough. Volumetric reconstruction may be facilitated by the helical movement and/or the 2D layout of the X-ray detector XD.

The scan box SB (data), on which more below at Figs 2, may be defined automatically or by user in embodiments, in particular by using a user input UI functionality as supported by facilitator system FS. Broadly, the scan box CB is a portion of 3D space in 3D image domain ER. It is thus a 3D object, but may be visualized in 2D view as a rectangle, hence the name scan box. The scan box defines the volume which is to be scanned in order to acquire projection data from which the volume of space demarked by the scan box is to be reconstructed, an in which the object of interest OI should be located.

The reconstructed volumetric imagery $V$ may be stored in a memory MEM or may be otherwise processed, as required. The reconstructed volumetric imagery *V* may be visualized by a visualizer VIZ. The visualizer VIZ may generate a graphics display of the volume or a given slice. The graphics display may be displayed on a display device DD. The visualizer VIZ may map a section of image volume V, or the image volume V as a whole, onto a grey value or a color palette. The visualizer VIZ may control, via suitable interfaces, video circuitry to effect display of the graphics display on the display device DD. In addition, or instead of so displaying, the reconstructed imagery *V* may be stored in a memory for later review or for other types of processing. Such a memory may include an image repository, such as a database (eg, PACS), or other (preferably) non-volatile data storage arrangement.

The reconstructed volume imagery *V* may be manipulated, for example by reformatting, to define cross-sectional planes that are different from the sectional plane defined by the imaging geometry. Such reformatting may allow medical user to better discern tissue type or anatomic details of the internals of the patient, in dependence on the medical purpose at hand, such as for diagnosis or preparation for some type of treatment, etc.

Whilst the following will be described with main reference to tomographic X-ray imaging, such as with an X-ray scanner of the doughnut type as shown in Fig. 1, or with C-arm scanners, or in any other design, the principles of scan box finding and definition as envisaged herein is not confined to X-ray based imaging, such as CT, but can also be practiced with benefit in other tomographic modalities, such as MRI, PET/SPECT, or any other where planning data need be established.

Broadly, the computing system CS may include, or may be capable of interfacing with, the said facilitator system FS. The facilitator system FS includes, maintains or manages computational capability, capable of assisting in a patient PAT positioning task. In particular, the facilitator system FS (simply referred to herein for brevity as *"the facilitator FS"*) helps ensuring that it is the object of interest OI, such as an organ, part thereof, etc., that is correctly positioned according to medical protocol prior to commencement of imaging, in particular of acquisition of projection data λ for a diagnostic scan, or for a locator scan. More particularly, the facilitator system FS localizes the object of interest OI as may be residing in the examination region ER (image domain) of the imager IA. For example, in some imagers of the tomographic type, such as CT or MRI, the examination region ER may be defined by a bore BR, in which the patient - or at least parts thereof - resides prior and during imaging. The object of interest IO may be localized in terms of positioning information, such as a co-ordinate, or a set of co-ordinates, etc., or in any other way or manner. The positioning information provided by the facilitator FS may define a current position of the object of interest OI, which may or may be the correct position. The correct position of the object is one according to user defined requirement, or according to imaging protocol, a set of rules drawn up for a given object of interest and the imaging purpose or objective at hand. The facilitator FS as envisaged herein is operable to establish the current position of the object of interest OI, thereby informing any suitable adjustments needed to ensure the correct position if achieved.

For example, an imaging geometry adjuster IGA of the imager IA may be operable to adjust, based on the positioning information provided by facilitator FS, the imaging geometry to ensure the said correct positioning of the object of interest OI. The imaging geometry in general pertains to the 3D configuration, or mutual spatial positioning in 3D space, of the acquisition equipment on the one hand, and the object of interest on the other. In particular, in X-ray based imaging settings, such as in CT settings, the imaging geometry may be at least partly defined by the mutual spatial 3D arrangement between certain components of the imager: for example the mutual spatial 3D arrangement may be one between the X-ray source XS, the X-ray detector XD, and the object of interest OI in particular. Imaging geometry may include re-orientation and/or repositioning of the acquisition equipment (any one or more of X-ray source and X-ray detector), or of the patient support PS, on which at least the object of interest OI resides in the examination region ER. The imaging geometry adjustment may further include control of a collimator (not shown) if present, and if needed. The imaging geometry adjustment may include changing position and/or orientation of any one or more of the above-mentioned components, including parts of whole of the acquisition equipment, the patient support (if any), collimator, etc.

The imaging geometry adjuster IGA may be operable automatically, semi-automatically, and indeed by manual adjustment by user. In the semi-automatic or manual embodiment, the current object of interest OI position as ascertained by facilitator FS may be displayed, for consideration by user, on a display device DD Optionally, such displaying may include displaying visual clues for suitable such adjustments to the user to put into practice, by operation of electro-mechanical actuator(s)/effector(s) through a control interface (e.g., joystick or the like), or by operation of suitable handles, hand-wheels, etc. In the semi-automatic setup, at least semi-assisted motorized support via a set of one or more such actuators AC is provided. Similar such actuators AC may be used in the or (fully) automatic setup, but this time such are instructed by the image geometry adjuster IGA based on the object OI position received from the facilitator FS, without intervention from human user. Imaging geometry adjuster IGA and facilitator FS, or either one of the two, may be integrated into operator console OC. Alternatively, at least parts of at least one of the two components (the imaging geometry adjuster IGA and facilitator FS) may be outsourced elsewhere, such as to a remote computing agent. However, following the principles of edge computing, and thus allowing for a more responsive operation of the system, the facilitator system FS may be arranged close to the data source, thus may be at least partly, if not fully, integrated into the imaging apparatus, such as into the operator console or into the broader supporting computing system CS associated with the imaging apparatus.

Figs 2 shows more details of positioning considerations of object OI as briefly mentioned above. Specifically, Fig. 2A illustrates the imager's bore BR, which defines the imaging domain/examination region ER. This is conceptually made up of an imaginary grid of grid points, such as voxel or "blobs", or other sets of primitive functions that define the image formation. The image domain is a portion of 3D space in which the patient support PS, if needed, is placed, and on it the patient PAT, in particular the object of interest OI.

The object of interest OI is sometimes referred to herein as a region of interest ("ROI") and relates to that part of the patient that is objective and purpose of the imaging to be conducted. The object is thus in general only a part of the patient. The terms object of interest and FROI are used interchangeably herein. The object of interest OI as shown in Fig. 2A may have a location relative to the imaging co-ordinate system $(X, Y, Z)$. This can be represented in a Cartesian co-ordinate system as is done here, with axes $X, Y,$ and $Z$ in 3D space. However, any other type of co-ordinate system may be used herein instead, with a different geometry, such as cylindrical, spherical, in polar co-ordinates, or any other can be used herein.

Referring now, for illustration, to the Cartesian imaging co-ordinate system, its $Z$ axis (the imaging axis) is run in general parallel to, or is coincidental with, the bore's BR central axis, with the bore forming a cylinder. If correctly positioned in the imager IA's bore/ examination region, this axis ought to be coincidental with patient's longitudinal axis when patient lies on patient support PS. The other two axes X,Y define in general the image co-ordinates, such as of the tomographic slices reconstructable from the projection data as acquirable after object positioning. In terms of the patient support, in general one of the imaging axes, say X, runs across the patient support, parallel to its shorter edge, whilst the other axis is perpendicular to the top TP of the patient support PS where the patient, or at least a part thereof, is received.

In contrast to the perspective view afforded by Fig. 2A, Fig. 2B shows a cross-sectional side elevation view along the imaging axis Z, which, in the said Fig. 2B, extends into the drawing plane of the Figure.

Fig. 2A illustrates a scan box SB, which is a subset of the examination region ER. Thus, in CT, MRI, or other tomographic modalities, the scan box SB may be a subset of the imager's bore BR. The said bore, or the examination region ER more generally, is a part of 3D space, made up of voxels as mentioned above. Ideally, the scan box should include the object of interest, sufficiently tightly, optionally with an applicable margin. Thus, the scan box is a subset of the image domain ER, BR, and yet ought to spatially include the space occupied by the object of interest. The scan box is a collection of all voxels for which image values are to be obtained. However, in order to define the scan box, the position of the object needs to be established, which is what the facilitator FS is configured to accomplish herein, and as will be explained in more detail below.

As can be seen, the current location/position of the object of interest OI may be referred to herein by co-ordinates $(x0,y0,z0)$, with the understanding that the position may also be defined by a group of co-ordinates, intervals of co-ordinates in either one or both or all directions $X,Y,Z$, as needed. For ease of illustration, only two of the three spatial co-ordinates $x0,y0$ are illustrated, with the third ($z0$) extending into the drawing plane of the Figure.

The object of interest is, in general, a 3D object such as one or both of the lungs, the liver, the diaphragm or the heart or respective portions of it, in particular a portion of the diaphragm, a lobe of the lungs, or parts of the heart's anatomy, such as its chambers etc. Thus, the object of interest has shape, size, position and possibly orientation.

The position of the object of interest, and hence the co-ordinates used to define the said position in any given co-ordinate system $(X, Y, Z),$ may thus refer to a salient point of the object of interest, such as to a centroid, to a center point, to an edge point, or any other, such as an extremity (e.g., tip point) of the relevant anatomical structure/feature, or may refer to any other feature, as pre-defined per protocol, such as a design parameter, or in any other way.

Once the position $p$ having co-ordinates $(x0,y0,z0)$ is known, the imaging geometry can be adjusted, if needed. For example, the imaging geometry may be so adjusted that an isocenter IS of the imager IA coincides with the said position $p$ of object of interest OI. This may include an adjustment as indicated schematically, such as movement/shift of the patient support PS. The imaging geometry adjustment may include a re-positioning of some or more imaging components, such as of patient support and or gantry, the acquisition equipment (detector XD and/or source XS). There may be a horizontal adjustment or re-positioning $\Delta x,$ and/or a vertical such adjustment $\Delta y$. In addition, or instead, there may be an adjustment $z0$ (not shown) in direction along axis $Z$ extending into the drawing plane, as needed. In addition or instead, the adjustment may include rotation(s) about any one, two, or all three of the axes $X,Y,Z$.

For example, such imaging geometry adjustment thus may include the re-positioning of gantry MG, SG that carries the X-ray source XS and/or detector XD, and/or may include using motorized actuators or manual implements to effect re-positioning of patient support table PS, to so ensure that the isocenter IS is, within applicable error margin, at the current position $p$ of the object of interest OI. As mentioned, the facilitator is operable to determine the location $p$ in the examination region of the object of interest, and this position information $p$ may then be used to take the appropriate action, such as imaging geometry adjustment, etc., as needed. In some cases, the patient is asked to move, etc.

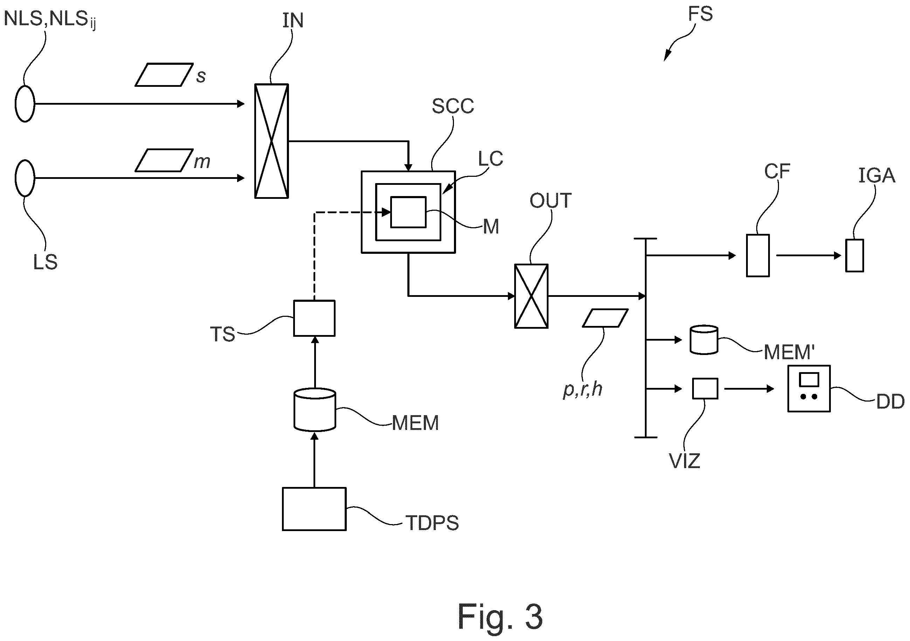

Operation of facilitator system FS is now described in more detail with reference to Fig. 3. Specifically, input data, to be described shortly herein in more detail, is received at one or more input ports or interfaces IN of the facilitator system FS.

A spatial characterizer component SCC is operable to characterize, based on such input data, one or more spatial characteristics of object OI. For example, the spatial characterizer component SCC may include a localizer component LC. The localizer component LC processes the input data and produces at output interface or output port OUT an estimate of the object of interest's position $p$ in the image domain ER, e.g. with reference to imaging coordinate system $(X,Y,Z)$. As mentioned, the position may be in terms of a single co-ordinate in the co-ordinate system, or in terms of a set of such co-ordinates, in terms of a geometric locus, etc., as required. For example, the position may be described by intervals in at least one, two or all three $(X, Y, Z)$ directions, Thus, such position information may be provided by defining a cuboid, sphere, ellipsoid, etc., which represents a sub-region/subset of 3D points in the examination region where the object is thought/estimated by facilitator FS to reside.

For example, such spatial sub-region for the object to reside, may be referred to as a scan box SB, although again, such set can have any shape, not necessarily a box shape. As shown in Fig. 2A, such scan box SB can be defined appropriately once the position is known, assuming an a priori known average size of the object of interest OI, or such size is estimated in addition to the object's OI position. Through a suitable communication interface, the output estimated position $p$ may be passed on to a control interface CI. Via the control interface, the imaging geometry adjuster IGA is instructed to effect the imaging geometry change(s). For example, a set of one or more actuators AC is operable, based on such instructions, to adjust the above mentioned one or more components of imager that define the imaging geometry.

The position information $p$ once computed may be stored in a memory MEM', or may be visualized by a visualizer VIZ on the display device DD. Such visualization may include an overlay graphics in 3D or 2D, against a visualization of the examination region imaging/imaging domain. As will be described in more detail, the localizer component LC may be driven by machine learning. Thus, the localizer component LC may include a trained machine learning model M. The machine learning model may have been trained by a training system TS, based on training data as may be held in a memory MEM.

The training data may have been provided by a training data provider system TDPS. The training system TS and the training provider TDPS are not necessarily part of the imaging arrangement IAR, or of the facilitator system FS, but are merely shown in Fig. 3 for the sake of completeness. Once the model has been trained on training data, which can be done in a preparatory phase (training phase), the so trained model can then be made available for the next phase (deployment or test phase), and the training data provider system TDPS or training system TS may no longer be needed as such. Having said that, periodical or irregular re-training of the model based on new training data as it emerges is certainly contemplated herein and can be done as needed. For example, at times, or quasi-continuously, the model is re-trained. New data provided over a certain period of time, e.g. during deployment time of the facilitator FS, is automatically used to re-train the model, based on the earlier trained model(s), to so obtain a sequence of consecutively trained model version(s) with improved performance.

Turning now to the input data $(s,m)$ shown on the upper left of Fig. 3, this may include spatial data or spatio-temporal data s, as may be collected/sensed by a suitable arrangement of sensors. In particular, the sensors may be non-line-of-sight sensors, that do not require a line-of-sight in respect of the object of interest OI. Such sensor readings s obtained by such non-line-of-sight sensors NLS may pertain to the patient, as he or she resides inside or outside the imager's bore BR. No ionizing radiation is needed for the operation of the non-line-of-sight sensors NLS, and indeed in some embodiments no radiation is required at all, although such (non-ionizing radiation) can still be used in other embodiments. In preferred embodiments, certain auxiliary data $m,$ such as imagery or other measurements, that do require line-of-sight for its operations are also envisaged as an additional input, as will be described in more detail below. In general, the non-line-of-sight sensors or plurality of such sensors NSL will be described shortly. In some embodiments, the non-line-of-sight sensors NLS provide spatio-temporal (spatial and temporal) signals in respect of the patient, in particular with respect to a "tell-tale" physiological activity of the object OI (embedded in the patient), whilst the line-of-sight measurement $m$, obtained by a different one or more sensors (more on which further below) provides outside morphological information of the patient as whole, and in whom the object of interest (e.g., an organ, etc.) is embedded. Thus, in brief, the input data preferably comprises spatial, in some embodiments spatio-temporal, data s, such as physiological data s, in relation to object of interest OI (generally within patient PAT), and morphological data $m$ of the said patient. The non-line-of-sight spatio-temporal data s may be referred to herein as the physiological signal, as such is indeed envisaged herein in preferred embodiments. However, in general the non-line-of-sight spatio-temporal data may not necessarily be physiological. In general, the non-line-of-sight spatio-temporal data is capable of being modulated by an activity (eg, motion or other change of state) of the object of interest, or of the surrogate object OI' (illustrated in Fig. 2B). Thus, the object of interest may be the originator of the activity, but other times the spatially related surrogate object is the originator. The said activity may or may not be physiological in nature. But, as said, for simplicity, and because a good number of embodiments are such, the non-line-of-sight spatio-temporal data s will be referred to herein as the physiological signal s as an example. Also, in the below, main reference will be made to the activity or signal s being one of the object of interest OI, with the understanding that this is a convenient shorthand, and that the activity and/or signal s may be in fact, and instead, one of the surrogate object OI'. Thus, the surrogate object OI' is used as a "surrogate" to spatially characterize the (actual) object of interest. In other cases, the surrogate object induces the activity in the object of interest, and the measured signal s pertains to the induced activity of the object of interest, and such signal will still be referred to herein as the physiological signal. If the measured activity is that of the surrogate object, the position or other spatial characterization of the object of interest OI can still be obtained, e.g., by machine learning. Machine learning learns the spatial relationship between the object of interest OI and the surrogate object OI'. Alternatively, a statistically determined spatial offset is applied to the position of the surrogate object to obtain the position of the object of interest OI. Any other calibration, or whichever type pf processing, can be used to ensure that the determined position/spatial characterization is that of the object of interest OI for the imaging task to be accomplished.

The physiological activity data s in respect of object of interest OI may be provided as sensor readings, as a collection of time series, or sensors NLS arranged at different locations *ij.* It is assumed herein that some of those spatio-temporal signal readings $s = s_{i,j}(t)$ are representative tell-tale signals of activity of the object of interest, in particular of physiological activity of the object of interest, which is thought to "betray" or "give away" the object's location in the imaging region. The "tell-tale" signals s*, as may be among the signals s received, or may be derived therefrom, have been found to correlate with the sought position *p* of the object of interest. Thus, collection of such signals allows localizing the object in particular, especially when the individual respective locations of the various sensors NLS are a priori-known, as is indeed assumed herein in some embodiments. In general, physiological activity may relate to motion, oscillation, deformation, rotation, etc., or to any other measurable activity carried out or entertained by an organ, group of organs, part of anatomy, etc., of human (or animal) patient in order to sustain normal vital function(s). Such physiological signals s may be derived from the set of spatio-temporal signals as received at input port.

For example, the non-line-of-sight sensor readings s are thought to relate to physiological activity, such as heart beats in case the heart is the object of interest, or breathing activity in case one or more of the lungs is/are the object of interest. In distinction to such physiological activity, two-channel input data is envisaged, wherein one channel corresponds to the said physiological activity data s, whilst the other channel pertains to the other additional input data provided by the line-of-sight sensor LS (or plural such sensors). Such sensor LS may relate to the patient's outside morphology. Such morphological data can be used in correlation with the physiological signal s for improved performance of the localizer component LC, e.g. better robustness, reliability, etc.

As mentioned, machine learning model implementation is envisaged herein in the main, but this may not necessarily be the case in all embodiments, where instead, or in addition, classical analytical signal processing approaches are used.

In some approaches, localizer analyses -- by spatio-temporal analysis -- the non-line-of-sight signals s in order to ascertain, and in particular to isolate, the "tell-tale" signals s*. Such signal s* may be correlated to the position *p* of the object of interest. In addition to establishing the position *p* of the object of interest, the spatial characterizer SC of facilitator FS may be capable of computing other spatial characteristics, such as shape *r* and/or extent/size *h* of the object of interest OI. Either one, or both of such shape *r* and/or size *h* in relation to object OI can be computed in addition to the position *p.* In some embodiments, of lesser interest herein, it may only be the size and/or shape that is computed.

As a matter of principle, the dual channel processing of the two data streams, comprising the non-line-of-sight sensor readings *s* and the line-of-sight readings *m,* is preferred herein.

The positioning information *p*, including the additional spatial characteristics of the objects such as the said shape *r* and/or size *h,* may be displayed, such as in a 2D or 3D visualization of the imaging region, thus allowing the user to effect adjusting the imaging arrangement accordingly, to ensure object is the field of view ("FOV")/scan box SB. In other cases, the position information may be displayed relative to a desired position. If there is a discrepancy, a call may be issued to patient to reposition themselves accordingly, so that the desired position is assumed. The call may be issued by human user, or automatically. The position information relative to a desired position may be displayed to the patient.

However, displaying of such information may not be needed, or is done in addition to automated processing of the position information *p*. In an automatic, such as an autonomous imaging or semi-automatic approach, the control interface CF instructs, based on the position information, actuator such as server motors, etc., to adjust the imaging geometry so that the object is within the scan box or FOV.

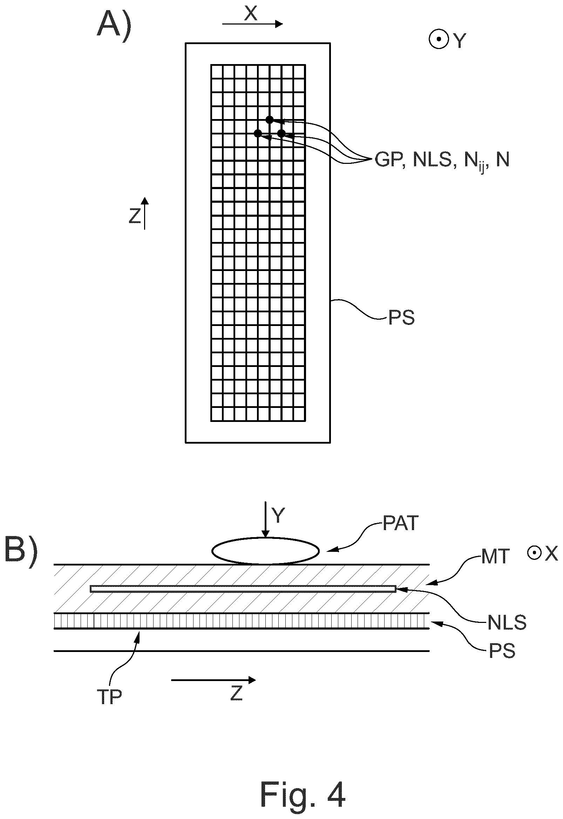

Referring now to Fig. 4, this shows details of a system of non-line-of-sight sensors NLS as envisaged herein in embodiments.

The non-line-of-sight sensors NLS are arranged in a matrix or 2D layout as shown in the plan view of Fig. 4A, with view along extending Y direction, perpendicular to the plane defined by the patient support PS. The non-line-of-sight sensors NLS form a 2D matrix spatial arrangement, with sensor nodes Nij spread out in suitably small spatial increments in *Z* and *X* direction as indicated by the double index notation (*i,j*). Each sensor node Nij may be individually addressable. Each sensor may furnish, for the spatial characterizer SCC to process, an individual reading *sij(t)* over a respective channel. Such sensors readings as furnished over the channels may be processed collectively by spatial characterizer SCC. On account of the 2D layout of the sensor nodes Nij, the readings form 2D map that map out spatial distribution of the readings. As the readings are changing over time, a spatio-temporal map or matrix *sij(t)* of such readings is obtained, and processed herein. Each sensor node's location *i,j* in the imaging coordinate system (*X,Y,Z*) may be known, e.g. by prior calibration. Processing of the readings and knowing the sensor nodes spatial location may allow localizing the object of interest OI.

As shown in Fig. 4B, the side elevation view along direction X or Y, the matrix of sensor nodes Nij may be integrated into a mattress MT of patient support PS as may be residing on the table-top TP of the patient support PS as envisaged herein in embodiments. The mattress MT may be removable for hygienic reasons. Alternatively, the matrix of sensor nodes Nij may be integrated into the support's PS table-top TP. For example, the table-top may be formed as an outer surface, such as a laminated layer, optional with some degree of flexibility such as a foamed layer, for better patient comfort, and it is within this outer layer that the matrix of nodes Nij are integrated. The layer may or may not be removable.

In either case, or in whichever manner, the integration of nodes Nij should be done in a manner, and the matrix of nodes should be so arranged, that the intended measurements can be delivered. In a proposed set-up, the sensor nodes Nij are pressure sensors capable of delivering through multiple channels, one channel per node in general, a set of spatially resolved pressure readings $s_{ij}$, where double index $(i,j)$ indicates the reading provided by the node Nij at its respective location ij. The pressure sensor nodes Nij may be arranged as piezo-electric components, spatially distributed or may be arranged as intersection points GP of optical fibers. Gravity is exerted by the patient as he or she lies on the patient support PS, and this causes pressure reading responses $s_{ij}$ according to the distribution of the patient's weight on the patient support PS, with the nodes integrated therein or otherwise therein arranged. Not only is the set of pressure readings $s_{ij}$ spatially resolved. There is in addition an over-time $t$ dependencies per node Nij, as this responds with readings at a certain sampling rate, or quasi-continuously, to yield spatial ("spatio")-temporal data $s_{ij}(t)$, with dependencies on time $t$ and node location $(i,j)$.

It has been found herein that the set of spatially resolved pressure readings, when analyzed over space and time in particular, allows for the extraction or identification of the said tell-tale physiological signals $s^*$. Specifically, it has been found that the physiological activity, if any, of the object of interest is reflected in a particular pattern in some of the readings provided by some of the pressure sensor nodes Nij. In general, the closer the nodes are to the object OI, the more pronounced is the modulation of the measured signals $sij$ by the physiological (or not) activity $\varphi$, and the more pronounced the pattern. For example, sensor nodes Nij that happen to lie under the object of interest, are likely to receive a stronger modulation. Because the location $(ij)$ of each sensor node Nij is known, so is then the position of the object of interest. Each sensor node ij whose signal is modulated in sufficient strength by the physiological (or non-physiological) $\varphi$ activity, defines a candidate location, and the set of all such candidate locations together then define as sufficiently "tight" region above which the object can be estimated to reside. A circumscribing geometric reference figures such as circle or rectangle or other may be passed through to the candidate locations. Spline curves may be used. This geometric reference figures may be taken to represent the location $p$. Alternatively, the geometric reference figure or the candidate locations are combined into a smaller number of parameters. For example, the candidate locations may be consolidated into a single parameter, such as coordinates of a reference point on the table-top TP plane of the patient support PS. For example, such reference point may be a point of the circumscribing geometric figure, such as a center point, barycenter, vertex, or any other.

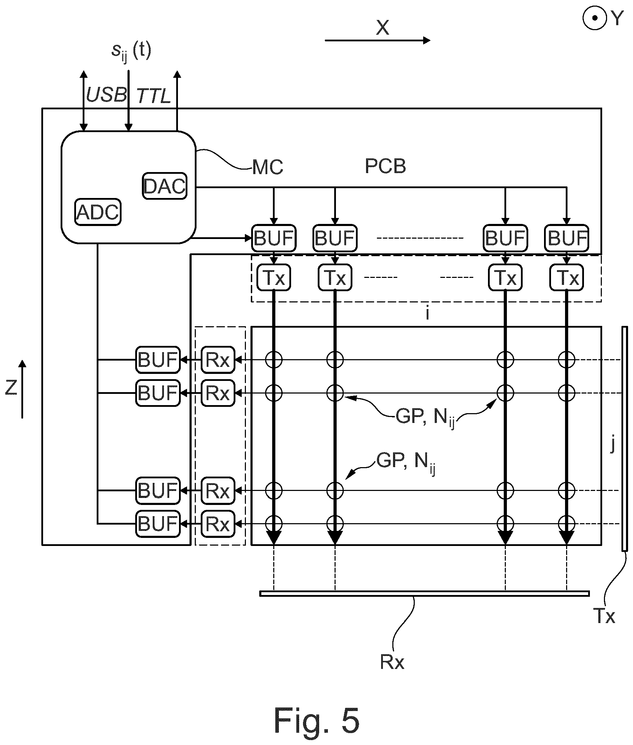

With reference to Fig. 5, this shows a schematic illustration of control elements of a matrix of pressures sensor nodes Nij as may be realized in some embodiments based on optical fibers. The optical fibers define channel. The optical fibers are run in a criss-crossing pattern, defining grid points GP where such fibers cross at different location $i,j$ distributed to form a part of the surface that is to receive patient's weight. The grid points GP represent spatially resolved sensor nodes Nij, each delivering a separate time series of pressure readings $s_{ij}(t)$ that can be read out through read-out circuitry, such as receiver circuitry Rx, that is made up of photosensitive detectors, one such detector for each channel.

The optical fibers as arranged in this grid layout to define the grid points GP, each gird point GP forming a different pressure sensor node, each responsive to deformation. The gravity force asserted by the patient residing on the patient support acts to deform the optical fibers at the grid points to different extents, depending on the weight distribution.

Some such nodes may receive a static pressure reading such as from prominent bone parts of the patient such as elbows, heels etc., whilst other nodes that happen to lie under the object of interest can detect in their time domain signal $s_{ij}(t)$ the (possibly periodic) physiological activity of the object of interest, thus revealing object's location as the location of the respective grid points GP and hence of the nodes Nij in the spatial co-ordinate system $(X, Y, Z)$, assumed herein to be known a priori as a matter of design choice. In particular, the physiological activity of the object OI may involve motion (rotation and/or translations, deformation), and such motion feeds through as pressure modulations in particular to the subset of sensors that happen to be proximal to the object of interest. The crossing optical fibers are arranged as a Bragg type interferometer. Thus, the internal structure of the optical fibers is deformed by gravity and the said modulations due to the object's physiological activity. Such fiber deformation causes a unique interference pattern which can be ascertained by sending light signals through transmitter electronics TX down the fibers. The change of interference pattern, and thus the change of light pattern, can then be received at the receiver RX electronics. The manner in which the interference pattern is changed correlates with deformation and thus with the pressure sustained locally at each grid point/node Nij. The light transmitter electronics TX is thus made up of light transmitters TX one for each fiber as shown in Fig. 5, and there is a similar arrangement for the light sensitive sensor, or other types of receivers RX, again one per channel line/optical fiber as shown.

Signal buffering BUF may be used. The receive-transmit circuitry Rx, Tx, the buffers BUF, etc., may be arranged at least partly on a printed circuit board PCB. The signals, in particular as received at the receiver Rx, may be optionally buffered at buffers BUF, and processed by a microcontroller or micro-processor. The micro-processor may operate digital-analogue conversion circuitry DAC or analogue-digital conversion circuitry ADC as shown in the Figure. In particular, analogue-digital conversion circuitry ADC may convert the received light pattern into pressure readings, thus into numerical form, which may be output at output port as the map of sensor readings, such as a universal serial bus USB for processing by the localizer component LC or other component of characterizer SCC as envisaged herein in embodiments. The providing of the output may be facilitated by logic circuitry, such as transistor-transistor logic TTL or other. The map of sensor readings may be stored in one or more memories as a matrix, or other suitable data structure, for processing by the spatial characterizer SCC. The map may not necessarily be so stored as whole at a given time, but parts of it may be stored at different times.

Instead of the arrangement based on optical fibers as shown in Fig. 5, a 2D matrix of piezo-electrical crystals may be used instead. Such crystals can be arranged in a discrete 2D pattern to so define the said pressure node points Nij.

However, the non-line-of-sight sensor arrangement shown in Fig. 5 is not confined to fiber objects or piezo-electronics, or indeed to pressure sensors. The non-light of sight sensor readings may be collected instead by radar, sonar/ultrasound, RF sensors, or any other measurement arrangements which may allow for capture of spatio-temporally resolved signals that can be correlated to a position, and optionally to size or shape of the object of interest OI, due to physiological activity of said object of interest OI (organ, part of organ, group of organs, tissue types, combinations thereof, anatomical feature, etc.) or of the surrogate object OI'.

In a more general setting, the localizer component may be part of the spatial characterizer component SCC which may include the said localizer component LC as one of its components. The spatial characterizer component SCC may optionally be configured to ascertain from the non-line-of-sight readings, in addition to position $p$, other spatial characterization of object/feature OI, such as shape $h$ and/or size r signature.

For example, as can be seen, the number of sensor nodes NIJ capable of responding to the physiological activity of the object of interest may be down to its size. All those nodes that are found by the spatial characterizer SCC to respond to the physiological signal can be used to define an area, and thus size, of the object of interest, with such area's shape corresponding to object OI's shape, at least in approximation and along some perspective view. Thus, in general, a group or subset of sensor nodes Nij may be found to be modulated according to the physiological activity, and a geometrical figure, such as a circle, ellipse, or rectangle, or other geometrical figure, etc., may be defined to enclose the so physiologically modulated sensor node locations ($i,j$), to so define not only location $p$ of object OI, but also shape $h$ and/or size $r$ of the object of interest.

Indeed, once the shape and/or size of the object is determined by the collection of appropriately responding nodes, the position of the object may be taken by computing a representative point for the shape/size figure so derived. For example, a circle may be generated by a geometry engine (not shown) of characterizer CSS to circumscribe the co-ordinates of the responding node locations. A center point of that circle may then be output as the position of the object of interest. However, this is merely an option. In other embodiments, the group of node locations found is combined or selected in any suitable manner to define the position $p$. The described reliance on shape and/or size, on the related geometrical figures, etc., is optional.

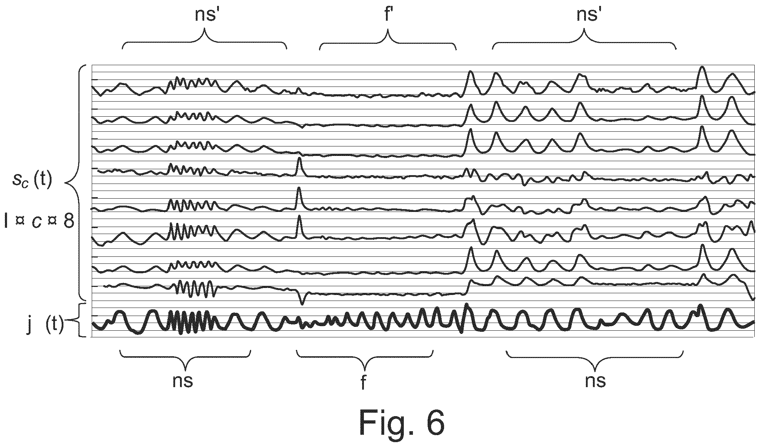

Fig. 6 shows proof-of-concept data. The data demonstrates that certain features of the physiological activity $\varphi$ (shown also as a signal in its own right) are reflected in pressure readings $s_j(t)$, which may have been taken with a sensor system as in Figs 4,5 or similar. For example, the true baseline physiological signal $\varphi(t)$ may be a time domain breathing signal, which is, as such, not usually available and there is no need for this in the present step. Whilst the signal s is referred to herein as the physiological signal, this is in distinction to the true physiological signal $\varphi(t)$. The latter signal $\varphi$ is capable to modulate the earlier signal s, as will be described now. Thus, the measured signal s may include modulated-into components/features s' that relates to, in particular is caused by, the (eg, physiological) activity of the object of interest (or by the surrogate object OI').

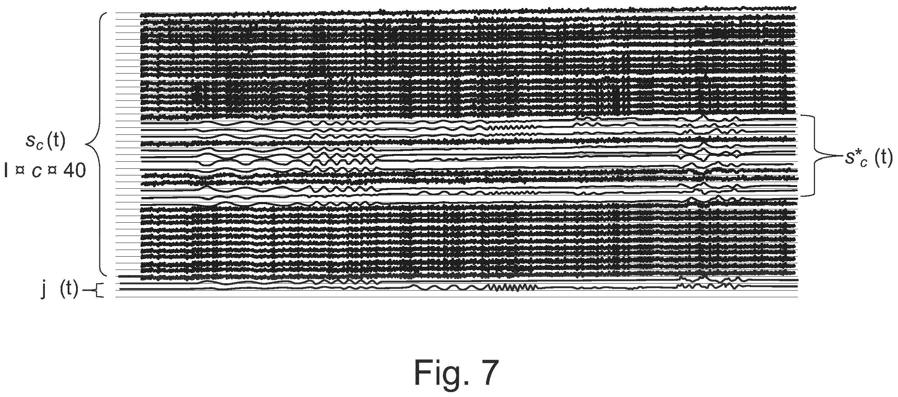

Turning first to the true physiological signal, this may comprise as features $s$' undulating sections $ns$', and in between a flat section $f$. Such may arise for example when the patient is breathing as shown in the first undulating section $ns$ to the left and is then asked for a breath-hold giving rise to the flat section $f$, which is then followed by a second undulating section $ns$' shown to the right, after the patient resumed breathing. This signature of the physiological signal can be reflected in some of the pressure readings $s_c(t)$ as collected for a number, in this case, eight channels for eight nodes such as those in the sensor matrix Figs 4,5, or other (in Figs 6 and 7 below, c is an index of a linearly list in which the double-index locations ($i,j$) of the matrix nodes Nij are assumed to be ordered for ease of notation - the ordering is arbitrary). The physiological signal $\varphi$ modulates the pressure readings as collected by some nodes and imparts corresponding undulating sections $ns$' with a flat section $f$ in between, thus reflecting the signature of the physiological signal very well.

The curves in Fig. 6 (and also in Fig. 7 below) represent an example of spatially resolved over-time sensor readings, which may not necessarily be pressure readings, but could be other signals such as acquired radar, laser, RF, or any other. Thus, Fig. 6 should be construed as a demonstration that the true physiological signal $\varphi$ can be reflected in a set of spatially resolved non-line-of-sight measurements, such as the pressure or weight readings s, or other suitable such signals such as amplitude or phase in wave signal a detectable by RF, US or radar sensors, in whichever way or manner they were acquired.

Fig. 7 is similar to Fig. 6 data, but in more detail for a more realistic pressure nodes layout in 5 x 8 grid points/channels, giving rise to 40 nodes distributed over the area which receives the weight of the patient such as the table-top, the mattress etc., as needed. Equi-distantly distributed nodes may be envisaged, but such may not necessarily be so in all embodiments, where an irregular layout could be contemplated although the equi-distant distribution pattern appears preferred. The inter-nodal distances determine the resolution of the measurements and a finer, more tightly arranged layout with small inter-nodal distances allows for a more accurate determination of any one of position $p$, size $r$ and shape $h$. The most suitable inter-nodal distances will depend on the case at hand. For example, for lung or heart imaging as envisaged herein in some embodiments, a layout with inter-nodal distances in the order or 1-5 cm may seem sufficient.

It can be seen in Fig. 7 that from the large number of pressure channels with pressure readings $s_c(t)$, it is only a handful of such readings $s^*_c(t)$ that are actually responding to the physiological signal, that are modulated into a corresponding pattern waveform or spectrum (in f-domain) patterns/features $s'$, according to the true physiological signal $\varphi(t)$.

The modulated pattern may be ascertainable in time domain but may also be ascertainable in frequency domain after a suitable harmonic analysis via transforms, such as Fourier transforms or other.

Turning now to the principles of operation of the spatial characterizer, in particular its localizer component LC, the data characteristics as exemplified above at Figs 5,6 may be used herein in different embodiments: non-machine learning embodiments and machine learning embodiments.

In non-machine learning embodiments of the spatial characterizer component SSC, in particular of its localizer component LC, this may be configured for spatio-temporal analysis of the over-time signals collected at the different nodes and received accordingly through their respective channels. The analysis may be for isolating, in particular, identifying the tell-tale signals s* which are those that have experienced sufficiently strong modulation according to the physiological activity $\varphi$ of the object of interest. The known positions of those candidate nodes Nij, which may be written herein as N*ij, may then be taken to define the sought position of the object of interest, the originator of the physiological activity $\varphi$. The multiple nodes positions may be collectively used as a definition of the object position as a set of points, or they me be combined as explained earlier into fewer coordinates, such as by averaging, or by defining circumscribing geometric figures (curves), and by suing salient one or more points of such figure/curve as the definition of the object position, such as a center point, corner point, etc. The spatio-temporal analysis by location component LC to identify the tell-tale readings s* from the set of pressure readings $sij$ may be done in time domain, possibly using Hilbert transform (that transforms into a time domain signal), filtering, etc. Waveform characteristics of the readings $sij$, etc. are compared against reference data that represents averages of physiological signals, etc., which are either known on medical grounds, or are determined experimentally or collected from existing medical records. Alternatively, the analysis is done in frequency domain after suitable transformation $T$ of the signals $sij$ by Fourier transform, Z-transform, Laplace transform, Wavelet transform or any other functional harmonic analyzer. In frequency domain, spectral characteristics of the transformed signals $T(sij) \rightarrow Sij$ may be compared against features of reference spectral characteristics spectral of physiological signals as may be stored as reference data. Again, data from existing medical records may be used after transformation into frequency domain.

The analysis in time or frequency domain may be based on a comparison of the acquired readings/transformed readings against the respective reference data. A policy may be used on which this comparison is based, such as thresholding and distance measure. The transformed Sij or raw readings sij may be analyzed compared based in on signal distance measure against the stored reference signature. A thresholding may be used to decide whether the respective signal is a tell-tale signal s * for the physiological signal $\varphi$. For example, if the frequency spectrum is sufficiently close to the reference spectrum stored, the respective node N*$ij$ can be assumed to be under the influence of the object of interest and thus sufficiently close. Thus, a selection of nodes N*$ij$ may be so isolated, and their locations $(i,j)$ can be used as an indicator for the object location, either as is, or by consolidating into fewer coordinate points (such as a single one), as described above by combination, averaging, definition of circumscribing figures, or by randomly choosing one of the node locations N*$ij$, etc.

In another embodiment, (temporal) cross correlation may be used to determine object size or position. Specifically, the signal $s$ may be correlated with the reference data, such the morphological data $m$.

For example, in some embodiments, the (temporal) cross-correlation between signals s$ij$(t) form one node and another node s$_{i'j'}(t)$ may be computed, to so determine signals which are similar. This is used to mark the nodes which bear similar signals and therefore give insight about the extent (size) of the organ.

In another embodiment, the (temporal) cross-correlation between a given node signal $sij(t)$, and the reference data is computed to determine whether the node signal can be considered close enough to the reference data, and can therefore be classified correspondingly. In this embodiment, the reference signal maybe another signal (either reference signal from a database, or signal from another sensor, including the morphological signal $m$ from the line-of-sight sensor LS). Signal demixing, e.g. using independent component analysis, etc., may be used for better performance.

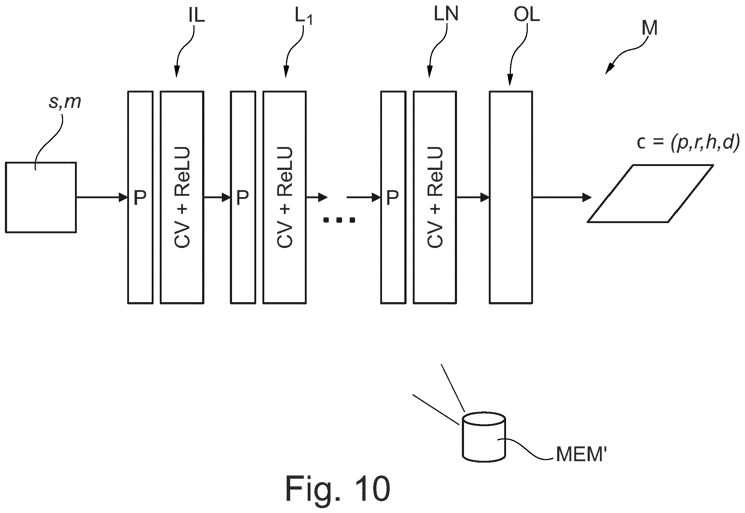

As an alternative to the above, analytical type processing, machine learning may be used instead by the spatial characterizer SCC, as will be described further below at Figs 10-12.

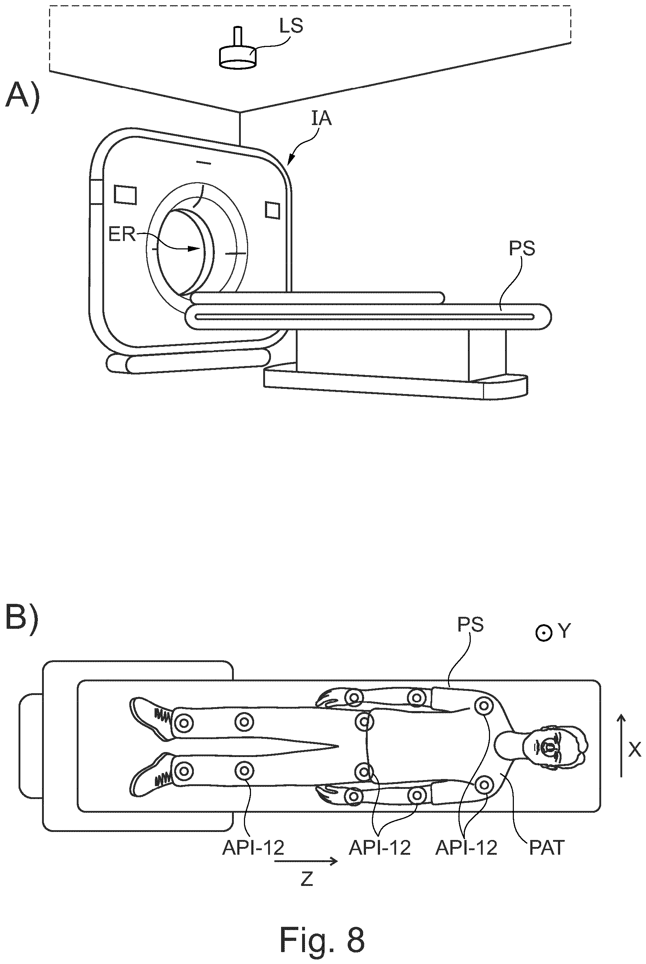

Turning now first to Fig. 8, this shows an illustration of a preferred embodiment where, in addition to the non-line-of-sight sensors NLS, one or more lines of sight sensors LS, such as overhead camera, may be used as illustrated in Fig. 8A.

This overhead camera may be arranged anywhere in the examination room or in the gantry of imager, or inside the bore etc. The camera may acquire patient imagery $m$ to provide a second stream of measurements that pertain to the physiology or morphology as seen from the outside of the patient. This camera may be a video camera. Thus, in some embodiments, the morphological measurements $m$ are time resolved (form a time series).

The sensor LS may be an optical camera, an infrared camera, depth sensing camera, radar, LIDAR, or may operate on any other non-ionizing based radiation. Image processing algorithms may be used by the spatial characterizer SCC or any other of its components, to process the received morphological measurements $m$ (such as camera pictures) to identify therein certain spatial anatomical key points or landmarks AP1-12. Some such landmarks are illustrated in the plan view of Fig. 8B, which shows the patient on the patient support, with certain salient anatomical key points AP 1-12, such as shoulders, elbows, wrists, hip joints or ankles, as needed.

The physiological measurements streams $sij(t)$, and the stream of morphological measurements/image data $m=m(t)$ as provided by line-of sight sensor LS may be processed together to ascertain the position, shape and/or size of the object of interest. Having said that, instead of being temporal time series data, the physiological measurement s and/or the morphological data $m$ may be static snapshot data. Thus, whilst in the following main reference is made to times series data, the principles described herein are of equal application to static data in the morphological or physiological channel, such support points, such as pressure points caused by head, feet, elbow, hip and shoulders. The coordinates of the identified landmarks AP1-12 in the morphological measurements $m(t)$ may be spatially registered with the coordinates of the sensor nodes Nij Thus, for some or each value $sij$ there is a corresponding image data $mij \in m$ (eg, pixel or image region) which may pertain to a particular anatomical landmark AP, segmentation thereof etc., and hence may provide additional clues on the object of interest's position.

Whether or not so registered, the morphological measurements $m(t)$ may be used in machine learning (ML) as a type of data driven regularization which may improve robustness of some type of ML algorithms. In the classical, analytical signal processing-based embodiment, the morphological measurements $m(t)$ may be used to filter out certain nodes, for better, more focused, more responsive processing. For example, the morphological measurements $m(t)$ may be used by localizer LC to exclude certain nodes from consideration, or may be given lesser weight, such as those nodes that happen to lie under prominent bones (heel, elbows), etc. and thus form static pressure points unlikely to be modulated by the physiological signal of interest. Equally, and in addition, or as an alternative, as the anatomical location of the object OI is known, the ascertained landmarks AP may be used to define certain "candidate" regions that are more likely than others to be proximal to the object OI. The localizer component LC may thus focus its processing (as per any one or more of the above described embodiments) on readings from nodes within those regions, or at least give higher weight to such readings, than to readings from outside the candidate regions.

As said, spatio-temporal (time series) data s is not necessarily needed herein in all embodiments. Static spatial data, such as the pressure sensor data, can be used instead to derive a silhouette of the patient, which may be sufficient to indicate gross positions of landmarks/anatomic key points AP (eg, anatomical features/), such as head, shoulders, thorax, hips, simply by analyzing the static spatial pattern of pressure. This static silhouette data $m$ can be combined with the morphological data $m$ form the line-of-sight sensor LS to improve detection accuracy and robustness in relation to the said landmarks. This approach may be especially useful in case of blankets covering the patient and hiding the body shape from the line-of-sight sensor LS. Thus, in this or similar scenarios, the static non-line-of sight data s complements the morphological data $m$. However, if needed for better accuracy or refinement, in addition, as mentioned, the so optionally complemented morphological data $m$, can be further combined with the spatio-temporal physiological signals $s$ to further predict or refine the position of object of interest OI, which may or may not be an internal organ or feature.

In embodiments, the in-image anatomic key points AP, or region(s) delineated by such in the imagery $m$, may be used as constraints. Only physiological signals $s$ that pertain to such anatomic key points AP as per the image $m$ are processed herein or given higher weights, or results from processing the physiological signals s are refined by using location information of landmark(s) AP as per the imagery $m$. Thus, in some examples, only a subset of physiological signals $s$ as determined based on the image data $m$ is processed into the spatial characterizing result, such as the said object of interest position. Whilst processing the physiological signals $s$ on their own, eg by temporal analysis in spatial or frequency domain (with subsequent back transformation into spatial domain) as mentioned above, allows some broad localization, the additional use of the morphological image data $m$ allows improving accuracy. For example, based on the (preferably registered) image data $m$, some rogue or artifact pressure readings in physiological data $s$ can be excluded. This is in particular because the spatial relationship between the object of interest OI and the landmark(s) AP can be assumed to be known in sufficient approximation, at least for a broad localization. The herein-described constraining or refining of the physiological signals $s$ based on image data $m$ may allow reducing a computational burden, with better responsiveness, and/or better accuracy. A converse approach may also be envisaged herein, where it is image data $m$ that is constrained based on physiological signals $s$.

Determining the said key points AP from the image data may be done by using any algorithm from computer vision, such as segmentations or other, either analytical or machine learning based, and such may be implemented by localizer component LC. This algorithm (eg ML model) for processing the image data $m$ may be different from the ML model M used for processing the physiological signals $s$ as such, but in some embodiments envisaged herein the same model may be used of processing both input data types.

In some embodiments, localizer component LC may function as a logic. It may compare information from sensor readings $sij$, such as per the temporal processing or machine learning processing described above, with counterpart information from image $m$ data: if information from both is consistent, the corresponding location ij is taken to be associated with object of interest OI's position. If there is a conflict, a scoring scheme may be used to quantify which information to "trust", that is, use as position information $p$ for object of interest OI. Either information derived from the physiological data $s$, or from image data $m$ may take precedence over the other. For example, if there is no visual occlusion at a particular location, image data $m$ may be given precedence over information obtained from physiological data s. If there is some visual occlusion, information obtained from physiological data $s$ may be given precedence. Other, or additional, such data reconciliation policy may be used.

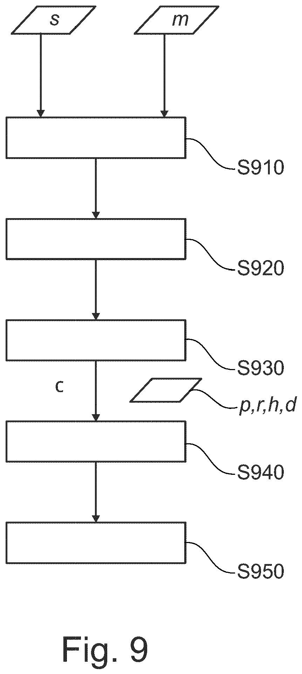

Reference is now made to Fig. 9 which shows a flow chart of a method of facilitating localizing an object of interest in an examination region 3D of an imaging apparatus such as of a CT, or other tomographic imaging modality, such as MRI or nuclear as needed. The method may be thought to implement operation of the spatial characterizer SCC, such as the localizer component LC, but the method may also be construed to represent a teaching in its own right. The proposed localizing may be used for adjusting an imaging geometry of the imaging apparatus.

At step S910 input data is received whilst the patient is in the examination room, in particular lies on the patient support PS or any other support as needed. The input data includes in particular, the physiological signal as acquired by a spatially distributed set of non-line-of-sight sensors, such as pressure sensors, or any other. In addition, the input data further includes morphological data provided by one or more line-of-sight sensor in relation to the patient.

The so received spatially resolved sensor readings may include over-time sensor readings from the spatially distributed sensor. The data may thus form spatio-temporal data. The input data is processed at step S920 into an estimate of the position of the object of interest in the examination region/image domain/bore of the imager.

At step S930 the so estimated position $p$ of the object of interest is then output and may be processed such as at step S940 to effect an imaging geometry adjustment, such as by (re-)positioning the patient, based on the estimated position. In this manner, it is ensured, or a likelihood is increased, that the object of interest is within the FOV of the imager IA. Alternatively, or in addition, the determined object position may be stored, displayed, or may be processed in any other way.

For example, based on the object of interest's position as determined at step S920, an imaging geometry of the imager may be adjusted, such as by re-positioning the patient support on which the patient lies and/or of the acquisition equipment, such as the source and/or detector in MRI or CT, as needed.

The use of the method in a fully automated imaging setting, such as in autonomous imaging is envisaged. In such settings, the imaging geometry change is effected automatically through suitable actuators and control interfaces but such autonomous imaging setting is not a requirement. Instead, manual intervention by the medical practitioner may still be envisaged herein, where the determined object position is used to suitably inform the user on the current position of the object of interest. In this embodiment, the object position information may be displayed suitably on a display device. For example, the object position may be displayed as an outline figure or as a hair-cross or other, within and against a visualization of the examination region/bore of the imager. Such displaying can also be done in the fully automated autonomous setting. The position data may also be used to issue other recommendation(s). For example, the recommendation may indicate to user whether and where to apply on patient PAT an additional sensing device, such as ECG leads or blood pressure sensing device. This recommendation could also comprise one or more parameters of contrast agent administration, such as volume of contrast agent needed, etc.

When the patient is suitably positioned based on the location information p, the imaging can commence at step S950 as needed. The above steps S910-S940 can be repeated until the correct positioning/imaging geometry is established.

The imaging at step S950 may include conducting a diagnostic scan or a locator scan. In the latter, low dose projection data (as compared to high dose in a diagnostic scan) is acquired to reconstruct the image $V$ as a surview image $V0$, which may be then used to plan the (later) diagnostic scan. Such locator scan may be needed in contrast agent assisted imaging protocols, where contrast agent is administered to boost image contrast when imaging for soft tissue objects.

It will be appreciated that the method described above in Fig. 9 is not necessarily confined to spatially resolved pressure readings. Other sensor readings that are capable of being modulated by physiological activity of the object of interest are also envisaged, such as radio frequency signals, radar, sonar, laser, or any other.

As said, in addition to the physiological signal s, the line-of-sight measurement $m$ may be received at input S910 that pertains to morphology as obtainable from the outside such as via an optical camera, video footage, depth sensing profiles, or by any other camera system. Thus, the method preferably functions as dual-stream processing, where physiological data s and morphology data $m$ is received and this is co-processed together by correlation or in another way to produce the position estimate.

As an extension still from the above, the step of determining the position of the object of interest based on input at step S930 may include determining further spatial characteristics $\chi$ of the object of interest, which may include in addition, or instead of such position, size $r$ and/or shape of the object $h$, and/or distance $d$ of object to landmark(s) or other object(s). Preferably however, it is shape and/or size of the object that is determined in addition to the object position to so fully inform positioning in imaging. It will be understood that the shape and/or size so determined may pertain to certain aspects of shape and/or size, such as spatial profiles as appear in certain projection view (such as along direction $Y$) of the object. Thus, the matrix layout of sensor reading may be able to ascertain the shape and/or size of the object in 2D outline (a silhouette), rather than in 3D. Having said that, some 3D information of the object OI may be recoverable to some extent from the 2D spatial data s by solving an inverse problem, especially if such data s is combined with other data, such as the line-of-sight data $m$ as is preferred herein.

In either case, the physiological signal is such that it should be capable of being suitably modulated by the physiological activity (the true physiological signal) of the object, and such activity should be reflected in some manner in the signal s obtained by spatio-temporal readings, such as by the presence of suitable signal signatures, patterns, and the like. Such spatio-temporal signatures for any one of position, size or shape may be a specific spectrum in frequency domain, but may also be ascertained in time domain by analysis of the wave form in any suitable manner by classical analytic approaches. Thus, such approach may be based on harmonic analysis, such as by applying any suitable harmonic analyzer such as Fourier transform, Wavelet transform, Z-transform, Laplace-transform, or an in-time domain transform such as Hilbert transform, by representation as analytical signal, or in any other. Reference data may be used that represents reference signatures and such are compared against the spatio-temporal signal $sij(t)$, to establish where the reading from a given node Nij is suitably modified, and hence is proximal to the object, thus revealing aspects of object's location, size or shape.

As to the size prediction of object, the spatially resolution of pressure sensors (which is a function of the above mentioned inter-nodal distance) should be configured sufficiently high. For example, if the nodes ae sufficiently close, rhythmical synchrony of sensed signals $sij$ acting together can be used to predict the size of the lungs, for example. The locations of those nodes Nij whose readings exhibit this synchrony, can be considered to demark the size of the object, at least in the X-Z plane. In a similar manner, rhythmical asynchrony can be used to determine the size and distance of e.g. the individual heart chambers. This is because heart contracts in two stages: in the first stage, the right and left atria contract at the same time, pumping blood to the right and left ventricles. Afterwards the ventricles contract together to propel blood out of the heart. Thus, in this and similar embodiments, details of the physiological functioning of the object (organ) are considered, and the estimation of size or position is based on such functioning. Specifically, it is considered how such physiological functioning are translated into observable signal pattens or properties, recordable by the pressure nodes. Location of nodes whose readings $sij(t)$ have pattern property are deemed to indicate size and/or position. Thus, for heart or lung(s), such signal pattern or property may be one of mutual synchrony or asynchrony between any two more node readings $sij(t)$ from different two or more nodes $Nij$. The extent to which there is such synchrony or asynchrony may be established by computing cross-correlation(covariance) or similar techniques from analytical time series analysis.

In an alternative embodiment it may be of interest to directly produce the distance between two organs based upon predetermined patterns in the output of the pressure sensors. Such distance can be automatically produced and used to automatically advice on e.g. radiation location and protocol.

For example, the strength or the pattern of the signal, as well as the number of activated pressure sensing nodes and the overall shape and size of the signal emitting region, can be indicators of the size of the organ.

However, the spatial characterizer step S930 is not necessarily based on such analytical signal processing, transforms, time series analysis, filtering, etc. Instead, the spatial characterizer step S930 may be based on machine learning. Thus, a trained machine learning ("ML") model may be used. In such approach, the model was trained in a prior training phase on training data. No specific analytical assumptions about the nature of the functional relationship between spatial characteristics and spatio-temporal sensor readings are needed. In particular, off-the-shelf models, such as neural network ("NN") type models, may be used, but need to be suitably trained on training data as described herein. In such training, parameters of the model are adjusted in an optimization procedure as administered by an ML training system TS.

One such suitable ML model is shown in Fig. 10 to which reference is now made. Fig. 10 shows an illustration of an architecture of a model, such as a neural network type model composed of a number of cascaded layers L$j$, in between input IL and output layer OL. More than one, in particular more than two such hidden layers may be used to form a deep learning architecture. The model in architecture includes nodes in the layers having parameters, The nodes are inter-connected such that nodes in one layer receive input from previous layers, and so on. Data flow may proceed from left to right with output from one layer passed on as input to the next layer, and so on, until the sought spatial characteristics $\chi$ of object such as position p and optionally size and/or shape emerge at output layer OL as a regression or classification result. The architecture and in particular the parameters $\theta$ of the model obtained through training may be stored in the memory MEM', and as may be used in deployment post-training, or may be used for testing on testing data, as needed.

Preferably, some or all of the hidden layers are convolutional layers, that is, they include one or more convolutional filters CV which process an input feature map from an earlier layer into intermediate output, sometimes referred to as logits. An optional bias term may be applied by addition for example. An activation layer processes in a non-linear manner the logits into a next generation feature map which is then output and passed as input to the next layer, and so forth. The activation layer may be implemented as a rectified linear unit RELU as shown, or as a *soft-max*-function, a sigmoid-function, *tanh*-function or any other suitable non-linear function. Optionally, there may be other functional layers such as pooling layers P or drop-out layers (not shown) to foster more robust learning. The pooling layers P reduce dimension of output whilst drop-out layer sever connections between nodes from different layers.

In embodiments, downstream of the sequence of convolutional layers, and upstream the output layer, there may be one or more fully connected layers (not shown), in particular if a regression result is sought. The output layer ensures that the output *p,h,r* has the correct size and/or dimension. In classification networks, the final layer may be a *soft-max* function layer.

The said convolutional NNs may be used, in combination with aspects of recurrent NN to better account for the over-time and spatial dependencies. Transformer type NNs using an encoder/decoder setup may be considered herein. Such transformer type NN have an attention mechanism that allows capturing, as trainable parameters, sequential contexts in the times series *sij(t)*. Some NNs type models allows processing of input in multiple channels, such as when processing color imagery in RGB channels. Such a multi-channel setup may be used herein when co-processing physiological and morphological input data (*s,m*) together as is mainly envisaged herein. The physiological and morphological input data may be presented as matrices which are treated as slices of a tensor. Each slice representing the combined data, the physiological and morphological channel, respectively. The convolution filters in the various layers are applied to such tensor data in cascaded fashion to so gradually merge data form the two channels into the desired output. Cross-channel convolutional filtering may be used.

Broadly, once suitably trained, the input data such as the physiological sensor readings spatially resolved s, optionally in in combination with the morphological measurements m, are received at input layer IL, and are propagated through the cascading layers thereby processed herein into feature maps as intermediate data in-between layers, and are then provided as final output at output layer OL as a regression or classification result, such as the position data *p* or, more generally, spatial characteristics data such shape *h* and/or size *r*, or distance *d* of object to landmark or other objects. The output data may be provided in any suitable format, such as one or more co-ordinates or groups of co-ordinates, subsets of voxels in image domain, etc, given the imaging co-ordinate system, etc.

Any type of ML model can be used herein that is capable of handling spatial data or, in some embodiments, spatio-temporal data. Thus, in particular the input layer IL may be configured to receive the physiological data as spatially resolved time series. Such data may be provided, stored and processed as a 3D tensor as mentioned above. Having said that, in some cases snapshot pressure readings (silhouette) may be sufficient, in which case representation in 2D matrix may be preferred as no time coordinate may be needed.

The above described ML solution are mainly intended for end-to-end processing, were the inputs *s*, or optionally (*s,m*), is regressed or classified directly into the desired spatial characterizer output $\chi$. However, such end-to-end processing is not a necessity herein. Instead, the ML arrangement may include a two stage pipeline, with the first stage including the ML model M for processing the input *s* or (*s,m*) into intermediate data, and this intermediate data is then processed analytically in the second stage into the desired output $\chi$. For example, each node signal sij(t) could be classified by such model M in the first stage into being sufficiently modulated by the physiology activity, or not. The type of physiology signal could be predicted by the model. The set of the nodes Nij whose readings are classified as sufficiently modulated then map out in 2D the size (extent) and/or position of the object OI.

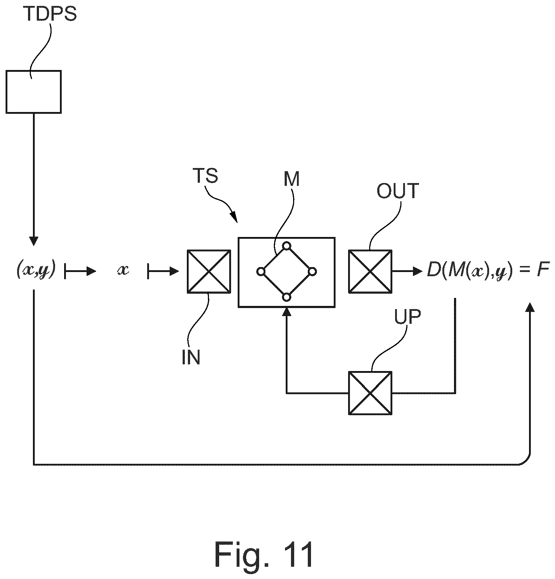

Fig. 11 shows a training system TS as may be used herein in embodiments. The training data (*x, y*) is in general different from the data seen by the trained model in deployment. The training data comprises x training input data *x* , and its related target *y* ("ground truth"). Such set-up in pairs of training data either (*x, y*) are particularly suited for supervised learning, but other learning schemes such as sub-supervised or non-supervised, reinforced can be used instead.

In training phase, an architecture of a machine learning model M, such as the shown CNN network in Fig. 4, is pre-populated with initial set of weights. The parameters $\theta$ of the model represent a parameterization $M^{\theta}$ of the model *M* (function). The parameters $\theta$ may include filter parameters of the convolutional operators CV, and/or other parameters. It is the object of the training system TS to optimize and hence adapt the parameters $\theta$ based on the training data. The training data may include a set of pairs $\{(x, y)^k\}$, where *k* indicates the respective instance of such pair $(x, y)^k: = (x_k, y_k)^k$. In other words, the training can be formulized mathematically as an optimization scheme where a cost function *F* is minimized although the dual formulation of maximizing a utility function may be used instead.

Assuming for now the paradigm of a cost function *F,* this measures the aggregated residue(s), that is, the summed error incurred between data estimated by the model and the targets as per some, or all, of the training data pairs k in a batch or overall training data:

$$argmin_{\theta} \, F = \sum_k D[\, M^{\theta}(x_k)\, , y_k\,] \hspace{3cm} (1)$$

In eq. (1), function $M()$ denotes the result of the model M applied to training input specimen $x_k$. The result will differ in general from the associated target $y_k$. This difference, or the respective residuals for each training pair $k$, are measured by a distance measure $D[\blacksquare, \blacksquare]$. Thus, the cost function $F$ may be pixel/voxel-based, such as the $L_1$ or $L_2$-norm cost function, or any other norm $L_p$. Specifically, The Euclidean-type cost function in (1) (such as least squares or similar) may be used for regressing measurement $x_k$ into an estimate of the object of interest position $M^{\theta}(x_k)$. When the model is to act as classifier, the summation in (1) is formulated instead as one of cross-entropy or Kullback-Leibler divergence or similar. Binary or multi-class classification may be envisaged herein for some embodiments, where a position estimation into spatial intervals (regions) is sufficient. Thus, the tabletop TP of support PS may be thought to be partitioned into regions ("cells") and the estimated output of model M is a classification, that is, an indication in which cell the object is situated, and is thus close or closest to.

The output training data $M^{\theta}(x_k)$ is an estimate for target $y_k$ associated with the applied input training sensor measurement $x_k$. As mentioned, in general there is an error between this output $M^{\theta}(x_k)$ and the associated target $y_k$ for each pair $k$. An optimization procedure such as backward/forward propagation or other gradient based method may then be used to adapt the parameters $\theta$ of the model M so as to decrease the residue for the considered pair $(x_k, y_k)^k$ or, preferably, a sum of residues for a batch (a subset) of such training pairs from the full training data set.

The optimization procedure may proceed iteratively. After one or more iterations in a first, inner, loop in which the parameters $\theta$ of the model are updated by updater UP for the current batch of pairs $(x_k, y_k)$, the training system TS enters a second, an outer, loop where a next batch of training data pairs $(x_{k+n}, y_{k+n})^{k+n}$, $(n=1,..,L)$ is processed accordingly. The structure of updater UP depends on the optimization procedure used. For example, the inner loop as administered by updater UP may be implemented by one or more forward and backward passes in a forward/backpropagation algorithm or other gradient based setup, based on the gradient $grad(F)$ of $F$. While adapting the parameters, the aggregated, for example summed, residues of all the training pairs in the given batch are considered. The aggregated residue can be formed by configuring the objective function $F$ as a sum of squared residues such as in eq. (1) of some or all considered residues for each batch. Other algebraic combinations instead of sums of squares are also envisaged. In general, the outer loop passes over batches (sets) of training data items. Each batch comprises plural training data items $k$, and the summation in (1) extends over the whole respective batch, rather than iterating one by one through the training pairs, although this latter option is not excluded herein.

In training, as administered by training system TS, the machine learning model learns a latent mapping $\mathcal{L}: s \to p$ that there is between the physiological readings on the one hand and the correct position of the patient on the other. Such mapping may be difficult to model analytically, but can be done by using the machine learning model and adapting its parameters based on the training data. A similar mapping is learned for the readings s vs shape $h$ or readings s vs size $r$ relationships. Thus, in general, the mapping to be learned may be written as $\mathcal{L}: s \to (p, r, h)$ In some embodiments with data-based regularization, the mapping is one of $\mathcal{L}: (s, m) \to (p, r, h)$. In particular, neural network models are known to be able to provide good approximates of such mapping.

The training data may be provided by a training data provider system TDPS. The training data includes, in particular, training input data $x$. The training input data includes examples or specimens of said physiological measurements which can be gathered from historical medical data as may exist in medical databases. The training data may further include related targets $y$, at least one such target $y$ for each given input $x$. Thus, in particular in supervised training setups, the training data may be conceptualized, stored, and processed as a set of data pairs $\{(x, y)^k\}$, where $k$ indicates the respective instance/specimen of such pair $(x, y)^k = (x_k, y_k)^k$. The target $y$ for each instance $k$ indicates the object position, given the readings $x$, such as pressure readings as explained above. The related position information $y$ may be represented in form such as the co-ordinates in the imaging coordinate system $(X, Y, Z)$, or in any other specifications that pertains to the position of the object when the historical readings were collected in prior exams, of a number of different patients in suitable variations across age, sex, BMI, etc.

However, at times it may be difficult to find such set of data pairs $\{(x, y)^k\}$ in existing databases. In such cases, field trials can be run to collect such data. A phantom, such as a human shaped phantom, with articulated joints etc., suitably realistic, may be placed in different positions on a patient support having the pressure nodes as described above to receive the weight of phantom. The phantom is repositioned to mimic different positions a patient may assume, thereby causing the recording of different pressure sensor readings which are then stored.

Suitable internal machinery or implements (such as pumps, etc.) inside the phantom may simulate physiological activity of heart and/or lung as needed, whilst phantom is in different such positions. For example, an oscillator may be used to simulate heartbeat, etc., issuing forth from within the phantom, thus imparting modulating signals on the pressure readings recorded by the underlying sensors.

The sensor data are recorded as spatial or spatio-temporal input data *x*, and are stored alongside ground truth/target *y*, which represents a characterization in space of the object of interest, such as position of the simulating implement within patient. This position is known. The phantom may be designed from a transparent material so that the position of the internal simulating implement may be easily ascertained by a human expert. The position information of the implement can be recorded as target data *y*, e.g., in terms of coordinates by a human expert. The human expert simply considers the phantom in the given position before them, and records the position of the simulating implement, and this is repeated for multiple phantom positions on the patient support. The human expert may provide the target position *y* as set of co-ordinates, for example through a suitable interactive graphical user interface as supported by a suitable computer viewing station for example. The viewing station causes a visualization of the examination region, e.g. as shown in plan-view to reveal a top view of the tabletop TP of the patient support PS. The expert visually examines the phantom on the tabletop and estimates the position of the simulating implement. His or her position estimate may then be recorded as an annotation by expert. To this end, the expert may use a pointer tool to designate the respective region of the implement, and this annotation is automatically associated with the current sensor readings as recorded. The sensor readings are in response to the given weight distribution of the phantom in the current position on the patient support, whilst the implement simulates the activity of interest.

Thus, expert may use an input device (such as the pointer tool) to designate the position of the respective model (simulating implement) of the organ or object of interest, given the position, orientation, arrangement, of the real phantom on the mattress or patient support. Thus, for each re-positioning of the phantom, the human expert may use the input device, such as a mouse or stylus, etc., to designate on screen or by touch screen action the respective position of the model of the object of interest that correlates with the phantom's position on the patient support. The position of the object of interest model may also be recoded in any other ways, such as by typing in coordinates, etc., the GUI based embodiments above being merely one example.

Thus, in embodiments, such as the ones described above, the training data provider system TDPS may include the sensor layout integrated in the patient/phantom support platform PS, optionally the phantom having the simulating implement, and a position recording tool, such as the computing system with GUI support, or any other. Thus, in embodiments, the training data provider system TDPS may include a computing system that allows the human expert to record, such as annotate, the respective object model positions within phantom with the phantom made to assume different position on the distributed layout if sensors. The recorded object model positions are associated with the corresponding sensor readings to so obtain the training data. Such association may be done automatically or may be mediated by the human expert.

The above training data providing procedure may be repeated with different phantoms of different sizes /weights. Instead of using such phantoms, the above procedure may be done instead with human volunteers, who (re-)position themselves on the matrix of sensors as integrated in the patient support, to so assume different positions, whilst the human expert notes the respective position of the object of interest using their anatomical/medical knowledge.

In this manner, either volunteer or phantom based, a suitably varied data set can be built up which can then be passed on for training by the training system TS. The training system TS may include any or more computing system with storage, and capable of running an ML training algorithm to train the model-based on the training data. The computing system may be capable for parallel computing. One or more GPUs or other specialist processor circuitry may be used to speed up the training.

Instead of physical a simulation by phantoms or volunteers/patients as described above, "digital phantoms" could be used to generate the training data by computer simulation based on bio-models (such as CAD, etc), combining internal organs, physiology, and body shape. Based on such data, the related non-line-of-sight data (and optionally the line-of-sight data) can be generated by randomizing CAD model parameters to obtain the training data. The advantage of such an approach is that a vast number of possible configurations can be generated at low cost, and the ground truth is obtained automatically in this manner.

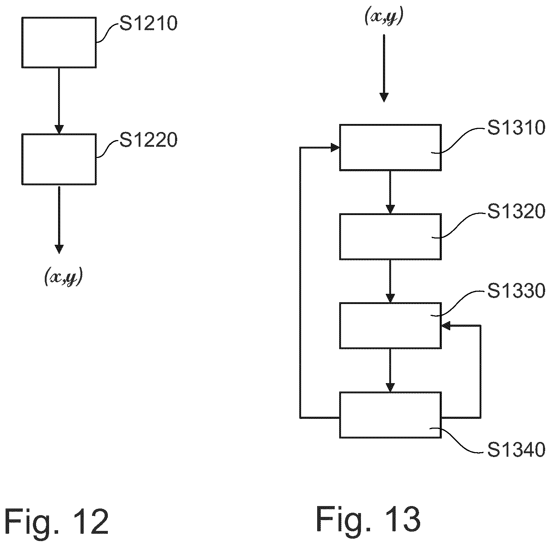

Reference is now made to Fig. 12 which shows a flow chart of a method of providing training data.

This may include step S 1210 where non-line-of-sight sensor readings as spatio-temporal data is recorded, whilst a volunteer or phantom assumes different positions on a matrix of sensors that respond with such readings.

In step S 1220 an associated object of interest position, such a position of an organ within the volunteer, or a position of a model of such object of interest within phantom, is recorded by a human expert. A graphical user interface may be used for such recordal. The sensor readings and the associated object of interest position are stored together as a training data pair. The above is repeated for any one or more of multiple phantoms, volunteers, positions, etc. to so obtain plural data pairs thus forming the training data set. In addition to annotating for object position, one or more distances to landmarks (such as the landmarks in AP1-12 in Fig. 8) may be annotated by expert, to so prepare training data that allows training model for predicting such distance(s).

It may also be possible to include the above described work-flow into everyday clinical practice where the object position is simply annotated by medical personnel in association with the respective sensor readings, to so built-up the training data in the required number (which may be in the hundreds or thousands), and in sufficient variation across any one or more of different objects of interest (eg, organs), patients of different bio-characteristics, and for different protocols as needed.

If the training data provider system TDPS relies on phantoms or volunteers/patients, body posture with and without knee pillow should be assumed. This is because such knee pillow can disturb the estimation of organ position, because the weight distribution will change considerably is has been found. Thus, to ensure better variation, pressure distributions should be record with phantom or volunteers using such knee pillows or supports for other body parts at times, whilst at other times no such support (eg knee pillow) is used.

In another embodiment, the training data provider system TDPS uses computer simulation as mentioned above. For example, the training data provider system TDPS uses a geometric shape model, such as CAD models, to simulate a wide variational range of body and organ extents, together with a typical range of varying respiratory and cardiac periodicities. The geometric CAD shape model may have articulations. Furthermore, the impact of these articulated body models onto matrix of pressure nodes is simulated. Indeed such computer simulation to obtain training data may be preferred over physical setups using volunteers/patients or phantoms.

In addition, or instead, generative ML models may be used to generate instances k of training data synthetically. For example, a GAN ("Generative Adversarial Networks") setup may be used.

If non-end-to-end models are to be trained, the ground truth data need to be modified accordingly.

Fig. 13 shows a flow chart of a method of training a machine learning model based on training data.

Such training method may include a step S 1310 where the training data is received. This may include historical patient data and/or the synthetically generated data, or data as generated by the method as per Fig. 12, or in whichever way obtained.

Broadly, based on the training data, parameters of the model are adapted. This adaptation may be done in an iterative optimization procedure., such as (1) or similar. The procedure is driven by a cost function $F$. Once a stopping condition is fulfilled, the model is considered trained.

In more detail, and with particular reference to a supervised training setup, at step S 1320, the training inputs $x_k$ in the current batch are applied to a machine learning model $M^\theta$ having current parameters $\theta$ to produce (respective) training output(s) $M^\theta(x_k)$.

A deviation, or residue, of the training output $M^\theta(x_k)$ from the respective associated target $y_k$, is quantified at S 1330 by cost function $F$. One or more parameters of the model are adapted at step S 1340 in one or more iterations in an inner loop to improve the cost function. For instance, the model parameters are adapted to decrease residues as measured by the cost function.

The training method then returns in an outer loop to step S 1310 where the next batch of training data is fed in. In step S 1320, the parameters of the model are adapted so that the aggregated residues, considered over the current and preferably over some or all previous batches, are decreased, in particular minimized. The cost function quantifies the aggregated residues. Forward- backward propagation or similar gradient-based techniques may be used in the inner loop. A dual formulation in terms of a maximization of a utility function is also envisaged.

Examples for gradient-based optimizations may include gradient descent, stochastic gradient. conjugate gradients, Maximum likelihood methods, EM-maximization, Gauss-Newton, and others. Approaches other than gradient-based ones are also envisaged, such as Nelder-Mead, Bayesian optimization, simulated annealing, genetic algorithms, Monte-Carlo methods, and others still.

A single such model may be trained, or multiple models may be trained, one for each protocol, organ or patient type, such as for children or adult, etc.

The training method in Fig. 12 is an example. Any form of training, such as clustering or in whichever way is envisage herein where a model is adapted based on training data.

The components of the facilitator system FS may be implemented as one or more software modules, run on one or more general-purpose processing units PU such as a workstation associated with the imager IA, or on a server computer associated with a group of imagers.

The above described can be generalized by reference to the surrogate object OI', which may differ from the actual object of interest OI. The object of interest OI is the purpose of the imaging, whilst the surrogate object OI' may not be so. The activity signal s may relate instead to the surrogate object. However, in this case, all the above described is still applicable as the ground truth training data still pertains to the spatial characterizing data $\chi$ of the object of interest. In addition still, in some causes the generator of the activity (eg, motion) is the surrogate object, and this induces related activity in the object of interest. In this cause, the spatio temporal measurement may pick up the activity of the surrogate object, or of the object of interest.

Alternatively, some or all components of the facilitator system FS may be arranged in hardware such as a suitably programmed microcontroller or microprocessor, such an FPGA (field-programmable-gate-array) or as a hardwired IC chip, an application specific integrated circuitry (ASIC), integrated into the imaging system facilitator system FS. In a further embodiment still, the facilitator system FS may be implemented in both, partly in software and partly in hardware.

The different components of the facilitator system FS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1.  System (FS) for localizing an object of interest (OI) for medical imaging, comprising:

    an input (IN) interface configured for receiving input data comprising i) a spatial map of non-line-of-sight sensor readings (s) from plural sensors (NLS, Nij) collectable in respect of a patient (PAT), and ii) further data ($m$)

providable by a line-of-sight based sensor (LS) in relation to the patient; and
a spatial characterizer (SC, LC) configured for determining, based on said spatial map of non-line-of-sight sensor readings (s) data and on the said further data ($m$), a current position ($p$) of said object of interest (OI).

2. System of claim 1, wherein said sensors (NLS, Nij) includes any one of: i) pressure sensors, ii) radar device, iii) RF device, iv) ultrasound device.

3. System of claim 1 or 2, wherein sensors are arrangeable in a spatial 2D pattern.

4. System of any one of the preceding claims, wherein the spatial characterizer (SC, LC) is operable to combine information from the spatial map of non-line-of-sight sensor readings with information from the said further data providable by the line-of-sight based sensor.

5. System of any one of the preceding claims, wherein the spatial characterizer (SC, LC) is based on a trained machine learning model (M).

6. System of any one of the preceding claims, wherein the spatial characterizer (SC, LC) capable of time-series waveform analysis or of spectral analysis of the said readings.

7. System of any one of the preceding claims, wherein the spatial characterizer (SC, LC) is capable of isolating in the sensor readings (s) a signal representative of an activity attributable to the object of interest (OI) or to a surrogate object (OI') spatially related to the object of interest.

8. System of any one of the preceding claims, wherein the spatial characterizer (SC) is capable of determining a size of the object of interest and/or a distance between said object of interest and another object of interest or landmark (AP1-12).

9. System of any one of the preceding claims, wherein the spatial map is a spatio-temporal map.

10. Arrangement comprising a system of any one of preceding claims, and further comprising any one of more of: i) a display device for displaying any one of: the determined position, size or distance ii) any one or more of the said sensors (NLS, Nij, LS), iii) the, or a, patient support, iv) the imaging apparatus, v) an imaging geometry adjuster (IGA) capable of adjusting an imaging geometry of the, or a, imagining apparatus, based on the said current position ($p$) of said object of interest (OI).

11. Training system (TS) for training, based on training data, the model of claim 5.

12. A training data provider (TDPS) capable of providing the training data for training of model by training system of claim 10.

13. Method for localizing an object of interest (OI) for medical imaging, comprising:

receiving (S910) input data comprising i) a spatial map of non-line-of-sight sensor readings (s) from plural sensors (NLS, Nij) collectable in respect of a patient (PAT) and ii) further data (m) providable by a line-of-sight based sensor (LS) in relation to the patient; and
determining (S920), based on said spatial map of non-line-of-sight sensor readings (s) and on the said further data ($m$), a current position ($p$) of said object of interest (OI).

14. A computer program element, which, when being executed by at least one processing unit, is adapted to cause the processing unit to perform the method of claim 13.

15. At least one computer readable medium having stored thereon the program element of claim 14, or having stored thereon the trained machine learning model (M) as per claim 5.

Fig. 1

Fig. 2

Fig. 3

A)

X →

⊙Y

GP, NLS, N$_{ij}$, N

Z ↑

PS

B)

↓Y

PAT

MT ⊙X

NLS

PS

TP

Z →

Fig. 4

Fig. 5

Fig. 6

Fig. 7

EP 4 696 224 A1

Fig. 8

Fig. 9

Fig. 10

EP 4 696 224 A1

Fig. 11

EP 4 696 224 A1

Fig. 12

Fig. 13

Europäisches Patentamt

European Patent Office

Office européen des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/205687 A1 (CAO JIANHUI [CN] ET AL) 4 July 2019 (2019-07-04)<br>* paragraph [0037] *<br>* paragraph [0055] *<br>* paragraph [0072] - paragraph [0074] *<br>* paragraph [0077] *<br>* paragraph [0079] - paragraph [0080] *<br>* paragraph [0082] *<br>* paragraph [0085] *<br>* paragraph [0095] - paragraph [0096] *<br>* paragraph [0110] *<br>* figures 1-11 * | 1-15 | INV.<br>A61B5/00<br>A61N2/00<br>A61B5/02<br>A61N5/10<br>G16H30/40<br>G16H40/63<br>G16H50/20 |
| A | US 2022/079514 A1 (MAIN IAN [CA] ET AL) 17 March 2022 (2022-03-17)<br>* paragraph [0040] - paragraph [0041] *<br>* paragraph [0086] - paragraph [0099] *<br>* paragraph [0107] - paragraph [0117] *<br>* figures 1-10 * | 1-15 | |
| A | US 2019/365256 A1 (DE GROOT KOEN THEO JOHAN [NL] ET AL) 5 December 2019 (2019-12-05)<br>* paragraph [0032] - paragraph [0040] *<br>* paragraph [0069] - paragraph [0074] *<br>* paragraph [0091] - paragraph [0093] *<br>* paragraph [0100] *<br>* paragraph [0108] *<br>* paragraph [0119] - paragraph [0121] *<br>* figures 1-10 * | 1-15 | **TECHNICAL FIELDS SEARCHED (IPC)**<br><br>A61B<br>G16H<br>A61N |

-/--

The present search report has been drawn up for all claims

| Place of search | Date of completion of the search | Examiner |
|---|---|---|
| The Hague | 21 September 2024 | Lai, Marco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 24 19 4607

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ROBERT GRIMM ET AL: "Markerless estimation of patient orientation, posture and pose using range and pressure imaging ; For automatic patient setup and scanner initialization in tomographic imaging", INTERNATIONAL JOURNAL OF COMPUTER ASSISTED RADIOLOGY AND SURGERY; A JOURNAL FOR INTERDISCIPLINARY RESEARCH, DEVELOPMENT AND APPLICATIONS OF IMAGE GUIDED DIAGNOSIS AND THERAPY, SPRINGER, BERLIN, DE, vol. 7, no. 6, 15 May 2012 (2012-05-15), pages 921-929, XP035132546, ISSN: 1861-6429, DOI: 10.1007/S11548-012-0694-5 * Sections "Materials and Methods" and "Experiments and Results" * * figures 1-4; tables 1-4 * ----- | 1-15 | |
| A | US 2021/121139 A1 (ANDREYEV ANDRIY [US] ET AL) 29 April 2021 (2021-04-29) * paragraph [0034] - paragraph [0036] * * figures 1-4 * ----- | 1-15 | |
| A | US 2020/110194 A1 (YOUNG STEVEN JAY [US] ET AL) 9 April 2020 (2020-04-09) * paragraph [0040] - paragraph [0043] * * paragraph [0058] - paragraph [0059] * * paragraph [0065] - paragraph [0068] * * figures 1-20 * ----- | 1-15 | TECHNICAL FIELDS SEARCHED (IPC) |

The present search report has been drawn up for all claims

| Place of search | Date of completion of the search | Examiner |
|---|---|---|
| The Hague | 21 September 2024 | Lai, Marco |

EPO FORM 1503 03.82 (P04C01)

EP 4 696 224 A1

## ANNEX TO THE EUROPEAN SEARCH REPORT
## ON EUROPEAN PATENT APPLICATION NO.

EP 24 19 4607

21-09-2024

| Patent document cited in search report | | Publication date | Patent family member(s) | | Publication date |
|---|---|---|---|---|---|
| US 2019205687 | A1 | 04-07-2019 | CN | 111542827 A | 14-08-2020 |
| | | | US | 2019205687 A1 | 04-07-2019 |
| | | | US | 2021012135 A1 | 14-01-2021 |
| | | | US | 2022222913 A1 | 14-07-2022 |
| | | | WO | 2019127385 A1 | 04-07-2019 |
| US 2022079514 | A1 | 17-03-2022 | NONE | | |
| US 2019365256 | A1 | 05-12-2019 | CN | 110049717 A | 23-07-2019 |
| | | | EP | 3551060 A1 | 16-10-2019 |
| | | | US | 2019365256 A1 | 05-12-2019 |
| | | | WO | 2018104359 A1 | 14-06-2018 |
| US 2021121139 | A1 | 29-04-2021 | CN | 110545730 A | 06-12-2019 |
| | | | EP | 3612099 A1 | 26-02-2020 |
| | | | JP | 2020517330 A | 18-06-2020 |
| | | | RU | 2019137095 A | 21-05-2021 |
| | | | US | 2021121139 A1 | 29-04-2021 |
| | | | WO | 2018192933 A1 | 25-10-2018 |
| US 2020110194 | A1 | 09-04-2020 | AU | 2019358908 A1 | 07-10-2021 |
| | | | EP | 3923802 A1 | 22-12-2021 |
| | | | US | 2020110194 A1 | 09-04-2020 |
| | | | WO | 2020076773 A1 | 16-04-2020 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021256921 A1

**Non-patent literature cited in the description**

- **T. M. MITCHELL**. Machine Learning. McGraw-Hill, 1997, 2